# EUROPÄISCHE PATENTSCHRIFT

(11) **EP 4 103 251 B1**
(45) Veröffentlichungstag und Bekanntmachung des Hinweises auf die Patenterteilung: **04.06.2025**
(21) Anmeldenummer: 21704737.2
(22) Anmeldetag: 08.02.2021
(51) Int. Cl.: A61M 5/20, A61M 5/315, A61M 5/32

(54) **AUTOINJEKTOR MIT EINER NADELSCHUTZHÜLSE**
AUTO-INJECTOR COMPRISING A NEEDLE PROTECTION SLEEVE
AUTO-INJECTEUR ÉQUIPÉ D'UN MANCHON DE PROTECTION D'AIGUILLE

(30) Priorität: 11.02.2020 CH 1492020; 11.02.2020 CH 1502020
(43) Veröffentlichungstag der Anmeldung: 21.12.2022
(73) Patentinhaber: Ypsomed AG, 3401 Burgdorf (CH)
(72) Erfinder: TSCHIRREN, Markus, 3400 Burgdorf (CH); HEINIGER, Lukas, 4932 Lotzwil (CH); WITTMANN, Jürgen, 3400 Burgdorf (CH); STETTLER, Peter, 3423 Ersigen (CH)
(74) Vertreter: Eugster, Monika Katharina
(86) Internationale Anmeldenummer: PCT/EP2021/052898
(87) Internationale Veröffentlichungsnummer: WO 2021/160540

(56) Entgegenhaltungen:
- EP-A2- 2 742 962
- WO-A1-2018/018165
- CH-A2- 711 240
- US-A1- 2010 274 185

## Beschreibung

Die Erfindung betrifft einen Autoinjektor, der oftmals auch als Autoinjektionsvorrichtung bezeichnet wird, mit dem ein in einem Produktbehälter enthaltenes Produkt nach dem Auslösen automatisch ausschüttbar ist. Bei dem flüssigen Produkt handelt es sich insbesondere um ein Medikament. Im Besonderen betrifft die Erfindung eine Signalisierungseinrichtung, die am Anfang und/oder am Ende der Produktausschüttung ein akustisches und/oder taktiles Signal abgibt, um den Anwender über den Beginn und/oder das Ende der Produktausschüttung zu informieren und/oder im Besonderen betrifft die Erfindung einen Autoinjektor mit einer Nadelschutzhülse.

Autoinjektoren sind im Stand der Technik bekannt. Ebenfalls sind Einrichtungen bekannt, die die Produktausschüttung signalisieren. Aus der DE 102008037310 A1 und der WO 2010/017650 A1 ist eine Injektionsvorrichtung bekannt, welche an der Kolbenstange ein Federelement aufweist, welches am Ende der automatischen Ausschüttung radial über Rippen am Gehäuse schnappt und den sogenannten "Endklick" bewirkt.

Weiter ist aus der EP2742962 eine Injektionsvorrichtung, insbesondere ein Autoinjektor bekannt, der einen sogenannten Startklick aufweist, welcher sich an der Kolbenstange abstützt und bei Auslösung des Autoinjektors sich von der Kolbenstange trennt und mittels einer Antriebsfeder in proximale Richtung verschoben wird um einen Startklick zu generieren.

Es ist eine Aufgabe der Erfindung, einen alternativen Autoinjektor anzugeben, wobei der Autoinjektor kostengünstig und betriebssicher ausgestaltet sein soll.

Die Aufgabe wird mit dem Autoinjektor nach Anspruch 1 gelöst. Vorteilhafte Weiterentwicklungen ergeben sich aus den abhängigen Ansprüchen, der Beschreibung und den Figuren.

Der erfindungsgemäße Autoinjektor weist ein Gehäuse und ein in dem Gehäuse angeordneten Produktbehälter auf. Bei dem Produktbehälter kann es sich insbesondere um eine Spritze handeln, welche einen Spritzenkörper aufweist, an dessen distalem Ende eine Injektionsnadel fest angeordnet ist. Der vorzugsweise zylindrische Spritzenkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere Medikament, durch die Injektionsnadel aus dem Produktbehälter ausgeschüttet wird. Der Spritzenkörper kann an seinem proximalen, d. h. hinteren oder der Injektionsnadel entgegengesetzten Ende einen Flansch, der auch als Fingerflansch bezeichnet werden kann, aufweisen. Eine derartig ausgebildete Spritze ist als Standardspritze erhältlich, so dass für den Autoinjektor nicht zwingend eine speziell angepasste Spritze entwickelt zu werden braucht. Der Kolben liegt dichtend an dem Innendurchmesser des Spritzenkörpers an.

Das Gehäuse ist vorzugsweise länglich und bildet die Längsachse des Autoinjektors. Das Gehäuse ist vorzugsweise hülsenförmig und/oder zylindrisch, insbesondere kreiszylindrisch. Der Produktbehälter ist in dem Gehäuse angeordnet. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In bevorzugten Ausführungen ist der Produktbehälter entlang der Längsachse unverschiebbar in dem Gehäuse aufgenommen, insbesondere mittels eines Produktbehälterhalters oder Spritzenhalters, der den Produktbehälter axialfest hält und axialfest mit dem Gehäuse verbunden, insbesondere verrastet ist. Bevorzugt steht die Nadelspitze über das distale Ende des Gehäuses in distale Richtung über. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Injektionsnadel aufweist, wobei die Nadel durch die Öffnung hindurchtreten kann. Die Nadelschutzhülse kann in ihrer Ausgangsposition in Bezug auf die Nadelspitze so angeordnet sein, dass die Nadelschutzhülse distal über die Nadelspitze steht oder dass die Nadelspitze distal über das distale Ende der Nadelschutzhülse steht. Die Nadelschutzhülse ist relativ zu dem Gehäuse aus ihrer Ausgangsposition in die proximale Richtung um einen Betätigungshub in eine betätigte Position verschiebbar, insbesondere in das Gehäuse verschiebbar, so dass die Nadel aus dem distalen Ende bzw. durch die Öffnung der Nadelschutzhülse hervortritt oder weiter hervortritt. Bevorzugt kann die Nadelschutzhülse um einen Nadelschutzhub aus der betätigten Position relativ zu dem Gehäuse in die distale Richtung in eine Nadelschutzposition verschoben werden, in der das distale Ende der Nadelschutzhülse distal über die Nadelspitze steht, um zu verhindern, dass nach erfolgter Verwendung der Vorrichtung bzw. nach erfolgter Produktausschüttung eine Verletzungsgefahr, die von einer freiliegenden Nadelspitze ausgehen würde, besteht. Die Nadelschutzhülse kann z. B. gegen die Kraft einer Feder, die als Nadelschutzfeder bezeichnet werden kann, in die proximale Richtung verschoben werden, wobei die Feder, die z. B. die weiter unten beschriebene zweite Feder oder eine hiervon separate Feder ist, die Nadelschutzhülse aus der betätigten Position in die distale Richtung, d. h. in die Nadelschutzposition verschieben kann. Der Autoinjektor kann ein z. B. federnd angeordnetes Verriegelungsglied aufweisen, welche die Nadelschutzhülse in ihrer Nadelschutzposition insbesondere in Bezug auf das Gehäuse verriegelt und ein Zurückschieben der Nadelschutzhülse in die proximale Richtung oder in das Gehäuse blockiert. Das Verriegelungsglied verriegelt die Nadelschutzhülse zumindest so, dass die Nadel nicht aus dem distalen Ende der Nadelschutzhülse hervortreten kann. Die Nadelschutzhülse kann z. B. aus der Nadelschutzposition nur soweit in die proximale Richtung verschoben werden, dass die Nadelspitze nicht aus dem distalen Ende der Nadelschutzhülse hervortritt.

Der Autoinjektor umfasst ferner ein Vortriebsglied, das zumindest während der Produktausschüttung auf den Kolben wirkt, insbesondere an dem Kolben anliegt, und eine erste Feder, die auf das Vortriebsglied wirkt wie z. B. sich insbesondere mit ihrem distalen Ende an dem Vortriebsglied abstützt. Das Vortriebsglied kann z. B. hülsenförmig sein. Ferner kann das Vortriebsglied eine Rippe oder mehrere Rippen, die z. B. im Bereich des distalen Endes des Vortriebsglieds angeordnet ist, umfassen. An der Rippe kann sich das distale Ende der ersten Feder abstützen. Die Rippe kann sich in proximale Richtung erstrecken. Die Rippe kann im Innern des Vortriebsglieds vorgesehen sein. Die Rippe des Vortriebsglieds kann z.B. an einer Innenseite des Vortriebsglieds vorgesehen sein. Die erste Feder ist vorzugsweise innerhalb des hülsenförmigen Vortriebsglieds angeordnet. Die Rippe des Vortriebsglieds kann zur Einstellung der Federspannung der ersten Feder dienen. Z.B. kann eine länger ausgebildete Rippe eine höhere Federvorspannung erzeugen. Die Rippe kann derart ausgebildet sein, dass die Federspannung der ersten Feder derart vorgespannt wird oder ist, dass das flüssige Produkt, insbesondere das gesamte Medikament in dem Produktbehälter ausschüttbar ist. Die Länge der Rippen oder der Rippe kann eine Vorspannung der ersten Feder so bestimmen, dass sie das Produkt aus dem Produktbehälter durch Verschieben des Vortriebsglieds um einen Ausschütthub (H_{A}) ausschütten kann. Die Rippe kann ferner zur Verstärkung des Vortriebsglieds, insbesondere des Teils des Vortriebsglieds an welchem die erste Feder abgestützt ist, dienen. Dadurch kann verhindert werden, dass das Vortriebsglied durch die Federkraft der ersten Feder beschädigt, insbesondere durchbrochen wird. In einer alternativen Ausführungsform können je nach Bedarf unterschiedliche Federn mit unterschiedlichen Federspannungen im Innern des Vortriebsglieds eingesetzt werden oder sein. Durch die Auswahl einer entsprechenden Feder, kann die Federspannung derart ausgewählt werden, dass das flüssige Produkt, insbesondere das gesamte Medikament in dem Produktbehälter ausschüttbar ist. Es können mindesten zwei Autoinjektoren vorliegen, wobei die entsprechende Länge der Rippen unterschiedlich ist, um eine gewünschte Federkraft zu haben. Alternativ können mindestens zwei Autoinjektoren vorliegen, wobei jeweils unterschiedliche Federn mit entsprechend unterschiedlicher Federspannung aufgenommen sind, um eine gewünschte Federkraft zu haben. Es kann insbesondere ein erster und ein zweiter Autoinjektor vorgesehen sein, wobei die Länge der Rippen des ersten Autoinjektors verschieden ist von der Länge der Rippen des zweiten Autoinjektors, und dass die Autoinjektoren ansonsten identisch sind. Die erste Feder ist vorzugsweise eine als Druckfeder wirkende Wendelfeder, die vorzugsweise aus Metall gebildet ist. Die erste Feder ist so stark vorgespannt, insbesondere im Auslieferungszustand des Autoinjektors, dass sie bzw. die in ihr gespeicherte Energie ausreicht, um das Produkt aus dem Produktbehälter durch Verschieben des Vortriebsglieds um einen Ausschütthub im Wesentlichen vollständig auszuschütten. Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied an dem Kolben bereits anliegt, entspricht der Ausschütthub des Kolbens dem Ausschütthub des Vortriebsglieds. Das proximale Ende der ersten Feder, die aufgrund ihrer Funktion auch als Ausschüttfeder bezeichnet werden kann, kann sich an dem Gehäuse oder einem gehäusefesten Element, insbesondere auch einem nur zeitweise zu dem Gehäuse axialfesten Element abstützen.

Erfindungsgemäß weist der Autoinjektor ein Signalglied, einen Signalanschlag und eine zweite Feder auf. Die zweite Feder kann eine auf das Signalglied entgegen der Ausschüttrichtung oder in die proximale Richtung wirkende Federkraft ausüben. Insbesondere kann sich die zweite Feder z. B. mit ihrem proximalen Ende an dem Signalglied abstützen.

Die zweite Feder kann z. B. eine als Druckfeder wirkende Wendelfeder sein, die sich mit ihrem proximalen Ende an dem Signalglied abstützt. Die zweite Feder ist vorzugsweise aus Metall gebildet. Die zweite Feder kann sich mit ihrem distalen Ende z. B. an dem Gehäuse oder einem gehäusefesten Element abstützen. Besonders bevorzugt stützt sich die zweite Feder mit ihrem distalen Ende an der Nadelschutzhülse oder einem Element, welches, insbesondere bei Verschiebung der Nadelschutzhülse relativ zu dem Gehäuse, mit der Nadelschutzhülse verschoben wird, ab. Beispielsweise kann das Element ein Schaltmodul oder eine Schalthülse, wie sie weiter unten beschrieben wird, sein. Das Element kann insbesondere kinematisch und/oder geometrisch zwischen der Nadelschutzhülse und dem distalen Ende der zweiten Feder angeordnet sein. Der Vorteil hierbei ist, dass die Nadelschutzhülse mittels der zweiten Feder aus ihrer betätigten Position in die Nadelschutzposition verschiebbar ist. Die Feder kann somit bevorzugt eine Doppelfunktion erfüllen, da sie zusätzlich die oben genannte Kraft auf das Signalglied ausübt.

Insbesondere im Auslieferungszustand oder während eines ersten Teilhubs des Ausschütthubs des Vortriebsglieds kann das Signalglied axialfest mit dem Vortriebsglied gekoppelt sein, so dass das Signalglied mit dem Vortriebsglied entlang der Längsachse und insbesondere relativ zu dem Gehäuse insbesondere in die distale Richtung verschiebbar ist. Durch die axialfeste Kopplung mit dem Vortriebsglied wird bewirkt, dass das Signalglied während des Verschiebens des Vortriebsglieds in die Ausschüttrichtung, insbesondere während der Ausführung des ersten Teilhubs des Ausschütthubs, mitgenommen und die zweite Feder gespannt wird. Während eines zweiten Teilhubs des Ausschütthubs ist es bevorzugt, dass die axialfeste Kopplung zwischen dem Signalglied und dem Vortriebsglied gelöst ist. Die axialfeste Kopplung zwischen dem Signalglied und dem Vortriebsglied ist somit lösbar. Wenn die axialfeste Kopplung zwischen dem Signalglied und dem Vortriebsglied gelöst ist - und insbesondere keine weiteren Kopplungen zwischen dem Signalglied und einem weiteren Glied, wie weiter unten beschrieben wird, bestehen - ist das Signalglied mittels der zweiten, vorgespannten Feder entgegen die Ausschüttrichtung und relativ zu dem Vortriebsglied und/oder dem Gehäuse beschleunigbar. Aufgrund der Mitnahme des Signalglieds durch das Vortriebsglied um den ersten Teilhub wird zwischen dem Signalanschlag und dem Signalglied ein z. B. sich entlang der Längsachse erstreckender Abstand, der insbesondere dem ersten Teilhub entspricht, gebildet. Die zweite Feder kann das Signalglied auf diesem Abstand beschleunigen, wodurch das Signalglied mit einer Geschwindigkeit auf den Signalanschlag auftrifft, so dass ein Impuls auf das Signalglied abgegeben wird, der ein akustisches (hörbares) und/oder taktiles (fühlbares) Signal erzeugt.

Der Signalanschlag kann von dem Gehäuse oder einem zumindest axialfest, vorzugsweise auch drehfest mit dem Gehäuse verbundenem Element gebildet werden. Beispielsweise kann dieses Element eine Verschlusskappe am proximalen Ende des Gehäuses sein und/oder das proximale Ende des Autoinjektors bilden. Die Verschlusskappe kann mit dem Gehäuse besonders bevorzugt formschlüssig, alternativ kraft- oder stoffschlüssig verbunden sein. Vorzugsweise ist das Element mit dem Gehäuse verrastet. Eine separate Kappe hat den Vorteil, dass die Montage der Vorrichtung erleichtert wird, wobei für die Montage zumindest ein Teil der Bauteile über das proximale Ende, das danach mit der Kappe verschlossen wird, in das Gehäuse eingebracht werden. Die Kappe kann einen Resonanzkörper bilden, wenn der Signalanschlag an der Kappe angeordnet ist, wobei durch die Gestaltung von Materialdicken und Formen der Kappe der Höreindruck des akustischen Signals in gewissen Grenzen verändert werden kann.

Das Signalglied weist in bevorzugten Ausführungen ein erstes, insbesondere federnd oder/und an einem federnden Arm angeordnetes, Eingriffsglied auf, welches in das Vortriebsglied, insbesondere in eine Ausnehmung des Vortriebsglieds lösbar eingreift. Hierdurch wird das Vortriebsglied axialfest mit dem Signalglied gekoppelt, wobei die axialfeste Kopplung zwischen dem Vortriebsglied und dem Signalglied gelöst ist, wenn das Signalglied, insbesondere das erste Eingriffsglied aus dem Eingriff mit dem Vortriebsglied, insbesondere der Ausnehmung des Vortriebsglieds ausgerückt oder herausgedrückt ist. Insbesondere wird das erste Eingriffsglied am Ende des ersten Teilhubs des Vortriebsglieds aus dem Eingriff mit dem Vortriebsglied gelöst.

Bevorzugt ist der Signalanschlag entlang der Längsachse des Autoinjektors so angeordnet, dass er in einer Flucht mit dem Signalglied angeordnet ist. Hierdurch wird erreicht, dass das Signalglied mit einer Bewegung entlang der Längsachse des Autoinjektors an dem Signalanschlag anschlägt.

In Ausführungsformen mit einer Nadelschutzhülse ist es bevorzugt, dass die Nadelschutzhülse auf die zweite Feder wirkt, wobei die Nadelschutzhülse für die Auslösung der Produktausschüttung aus ihrer Ausgangsposition relativ zu dem Gehäuse und entlang der Längsachse des Autoinjektors in die proximale Richtung, d. h. entgegen die Ausschüttrichtung verschiebbar ist, insbesondere um den Betätigungshub. Hierdurch wird die zweite Feder gespannt und vorzugsweise auch die Produktausschüttung, insbesondere die Bewegung des Vortriebsglieds in die Ausschüttrichtung, ausgelöst. Die Nadelschutzhülse wird vorzugsweise dadurch aus ihrer Ausgangsposition um den Betätigungshub in ihre betätigte Position bewegt, dass ihr distales Ende an die Einstichstelle des Patienten angedrückt wird, wobei das Gehäuse relativ zu der Nadelschutzhülse in Richtung Einstichstelle verschoben wird, so dass die Nadelschutzhülse den Betätigungshub relativ zu dem Gehäuse ausführt. Hierbei wird auch die aus dem distalen Ende der Nadelschutzhülse hervortretende Nadel in die Einstichstelle eingestochen. Nach der erfolgten Produktausschüttung, insbesondere nach z. B. einer kurzen Wartezeit, wie z. B. 3 bis 10 Sekunden, nach dem mittels des Signalglieds das Signal erzeugt wurde, wird der Autoinjektor von der Einstichstelle abgenommen, wodurch die Nadelschutzhülse relativ zu dem Gehäuse aus ihrer betätigten Position um den Nadelschutzhub in die Nadelschutzposition verschoben wird, insbesondere mittels der in der zweiten Feder gespeicherten Federenergie. Durch das Abnehmen des Autoinjektors von der Einstichstelle wird auch die Nadel aus der Einstichstelle gezogen.

In bestimmten Ausführungsformen kann kinematisch zwischen der zweiten Feder und der Nadelschutzhülse ein Schaltmodul angeordnet sein, wobei das Schaltmodul von der Nadelschutzhülse in die proximale Richtung mitgenommen wird, wenn die Nadelschutzhülse aus ihrer Ausgangsposition in die proximale Richtung oder in die betätigte Position verschoben wird, und die Nadelschutzhülse in die distale Richtung verschiebt, wenn die auf das Schaltmodul wirkende Feder das Schaltmodul in die distale Richtung verschiebt. Das Schaltmodul oder ein Teil davon, wie z. B. eine Schalthülse, kann mit der Nadelschutzhülse einteilig sein oder z. B. formschlüssig verbunden, wie z. B. verschnappt, sein oder lose an der Nadelschutzhülse anliegen. Das Schaltmodul kann ein einziges Teil sein oder mehrere Teile umfassen, wobei ein mehrteiliges Schaltmodul zumindest die Schalthülse und eine Sperrhülse aufweisen kann. Die Sperrhülse kann relativ zu der Nadelschutzhülse und/oder Schalthülse z. B. entlang der Längsachse verschiebbar sein.

Beispielsweise können sich die zweite Feder an der Schalthülse und die Schalthülse an der Nadelschutzhülse abstützen. In alternativen Ausführungen können Schalthülse und Nadelschutzhülse axialfest und vorzugsweise drehfest verbunden sein, wobei die zweite Feder an der Schalthülse abgestützt sein kann. In weiteren alternativen Ausführungsformen können Schalthülse und Nadelschutzhülse einteilig ausgebildet sein, wobei die zweite Feder an der Schalthülse abgestützt sein kann. Zwischen der Sperrhülse und der Schalthülse kann ein z. B. unidirektional wirkendes Verriegelungsglied, welches vorzugsweise das oben genannte Verriegelungsglied, welches die Nadelschutzhülse in ihrer Nadelschutzposition verriegelt, vorgesehen sein, das z. B. von der Sperrhülse gebildet wird und in die Schalthülse, insbesondere in eine Ausnehmung, eingreift. Das Verriegelungsglied ist vorzugsweise so ausgestaltet, dass die Schalthülse während ihrer Bewegung relativ zu dem Gehäuse in die proximale Richtung die Sperrhülse über das Verriegelungsglied mitnimmt, insbesondere während der Bewegung der Nadelschutzhülse aus ihrer Ausgangsposition in die betätigte Position, und während ihrer Bewegung relativ zu dem Gehäuse in die distale Richtung relativ zu der Sperrhülse in eine Sperrposition verschoben wird, insbesondere während der Verschiebung der Nadelschutzhülse aus ihrer betätigten Position in die Nadelschutzposition, wobei in der Sperrposition das oder ein anderes Verriegelungsglied, wie z. B. das weiter oben genannte, eine Bewegung der Schalthülse relativ zu der Sperrhülse in die proximale Richtung sperrt. Hierdurch wird vorteilhaft verhindert, dass die Nadelschutzhülse aus ihrer Nadelschutzposition für eine erneute Freigabe der Nadelspitze in das Gehäuse zurückgeschoben werden kann.

Z. B. kann die Schalthülse eine erste Ausnehmung aufweisen, in welche das Verriegelungsglied der Sperrhülse lösbar eingreift, wenn die Nadelschutzhülse aus ihrer Ausgangsposition in ihre betätigte Position verschoben wird. Z. B. kann die Schalthülse eine zweite Ausnehmung aufweisen, in welche das Verriegelungsglied oder ggf. das andere Verriegelungsglied eingreift, wenn die Nadelschutzhülse in ihrer Nadelschutzposition ist. Die erste und zweite Ausnehmung können vorzugsweise mit einem Abstand, der in etwa dem Nadelschutzhub entspricht, zueinander entlang der Längsachse angeordnet sein. Selbstverständlich ist auch eine Umkehr der Anordnung der Ausnehmungen und des Verriegelungsglieds oder der Verriegelungsglieder möglich, d. h., dass das zumindest eine Verriegelungsglied an der Schalthülse und die mindestens eine Ausnehmung, d. h. die erste Ausnehmung und ggf. die zweite Ausnehmung an der Sperrhülse gebildet sein können.

In alternativen Ausführungen kann die Schalthülse eine erste Ausnehmung aufweisen, in welche das Verriegelungsglied oder Verriegelungsglieder der Sperrhülse lösbar eingreift, wenn die Nadelschutzhülse aus ihrer Ausgangsposition in ihre betätigte Position verschoben wird. Das Verriegelungsglied der Sperrhülse kann an das proximale Ende der Schalthülse anschlagen, wenn die Nadelschutzhülse in ihrer Nadelschutzposition ist. Selbstverständlich ist auch eine Umkehr der Anordnung der Ausnehmung und des Verriegelungsglieds möglich.

Das Verriegelungsglied und ggf. das andere Verriegelungsglied können federnd, insbesondere jeweils an einem federnden Arm, angeordnet sein. Vorzugsweise kann die Schalthülse die Sperrhülse umgeben und/oder führen.

In bevorzugten Ausführungen kann das Signalglied ein zweites Eingriffsglied aufweisen, welches durch die Ausrückbewegung des ersten Eingriffsglieds, mit der das erste Eingriffsglied aus dem Vortriebsglied ausrückt, in einen, insbesondere axialfesten, Eingriff mit der Nadelschutzhülse oder dem Schaltmodul, insbesondere der Sperrhülse, bewegbar ist. Das erste Eingriffsglied und das zweite Eingriffsglied des Signalglieds sind bevorzugt so aufeinander abgestimmt, dass das zweite Eingriffsglied bereits, vorzugsweise axialfest, in die Nadelschutzhülse oder das Schaltmodul eingreift, wenn das erste Eingriffsglied noch nicht vollständig aus dem Eingriff mit dem Vortriebsglied gelöst ist. Hierdurch wird vorteilhaft zuverlässig verhindert, dass das erste Eingriffsglied bereits aus dem Eingriff mit dem Vortriebsglied gelöst ist, wenn das zweite Eingriffsglied noch nicht in dem Eingriff mit der Nadelschutzhülse oder dem Schaltmodul, insbesondere der Sperrhülse ist. Die Nadelschutzhülse oder das Schaltmodul, insbesondere die Sperrhülse, kann eine z. B. weitere Ausnehmung aufweisen, in welche das zweite Eingriffsglied des Signalglieds, z. B. für die axialfeste Kopplung zwischen dem Signalglied und dem Schaltmodul, insbesondere Sperrhülse, oder der Nadelschutzhülse, eingreift. Das Vortriebsglied kann eine Ausnehmung aufweisen, in welche das erste Eingriffsglied für die axialfeste Kopplung zwischen Vortriebsglied und Signalglied eingreift. Vorzugsweise sind das erste Eingriffsglied und das zweite Eingriffsglied an einem gemeinsamen elastischen Arm gebildet, wobei das erste Eingriffsglied, z. B. radial, zu der Längsachse hin und das zweite Eingriffsglied, z. B. radial, von der Längsachse weg weisen. Das erste und zweite Eingriffsglied können, vorzugsweise radial zwischen dem Vortriebsglied und der Nadelschutzhülse oder dem Schaltmodul, insbesondere der Sperrhülse angeordnet sein.

Insbesondere werden während des Ausschütthubs des Vortriebsglieds, insbesondere am Ende des ersten Teilhubs, das erste Eingriffsglied des Signalglieds aus dem Eingriff mit dem Vortriebsglied gelöst und, vorzugsweise gleichzeitig, das zweite Eingriffsglied des Signalglieds in den Eingriff mit dem Schaltmodul oder der Nadelschutzhülse gebracht, insbesondere mit einer Bewegung quer zur Längsachse. Insbesondere kann das Vortriebsglied durch seine Bewegung in die Ausschüttrichtung das erste Eingriffsglied aus der Ausnehmung des Vortriebsglieds und das zweite Eingriffsglied in die Ausnehmung der Nadelschutzhülse oder des Schaltmoduls, insbesondere der Sperrhülse, drücken.

In besonders bevorzugten Ausführungen kann die Nadelschutzhülse oder das Schaltmodul, insbesondere die Sperrhülse, das erste Eingriffsglied des Signalglieds in dem Eingriff mit der Ausnehmung des Vortriebsglieds halten, wobei die Ausnehmung für das zweite Eingriffsglied des Signalglieds durch Verschieben der Nadelschutzhülse aus ihrer Ausgangsposition in ihre betätigte Position bezogen auf die Längsachse zu dem zweiten Eingriffsglied hin verschoben wird, wobei die Ausnehmung in der betätigten Position der Nadelschutzhülse, insbesondere in dem Moment, an dem der Ausschütthub freigegeben wird, mit einem Abstand entlang der Längsachse, die in etwa dem ersten Teilhub des Signalglieds entspricht, zu dem zweiten Eingriffsglied angeordnet ist. Das für den Ausschütthub durch die Betätigung der Nadelschutzhülse freigegebene Vortriebsglied ist dann um den ersten Teilhub in die Auschüttrichtung bewegbar. Vorzugsweise wird das erste Eingriffsglied in dem Eingriff mit dem Vortriebsglied durch den Innenumfang der Nadelschutzhülse oder des Schaltmoduls, insbesondere der Sperrhülse gehalten, an der bzw. dem das zweite Eingriffsglied anliegt. Das zweite Eingriffsglied befindet sich am Ende des ersten Teilhubs bezogen auf die Längsachse auf der gleichen Position wie die Ausnehmung, wodurch das zweite Eingriffsglied in seine Ausnehmung einrücken und das erste Eingriffsglied aus seiner Ausnehmung ausrücken kann.

Der Ausschütthub des Vortriebsglieds kann insbesondere zwei Phasen umfassen, nämlich den ersten Teilhub und den zweiten Teilhub. Während des ersten Teilhubs ist das erste Eingriffsglied des Signalglieds in dem axialfesten Eingriff mit dem Vortriebsglied und das zweite Eingriffsglied des Signalglieds aus dem axialfesten Eingriff mit der Nadelschutzhülse oder dem Schaltmodul, insbesondere der Sperrhülse. Während des zweiten Teilhubs des Ausschütthubs ist das zweite Eingriffsglied in dem axialfesten Eingriff mit der Nadelschutzhülse oder dem Schaltmodul, insbesondere der Sperrhülse, wobei das erste Eingriffsglied aus dem Eingriff mit dem Vortriebsglied ist, wodurch vorteilhaft bewirkt wird, dass das Vortriebsglied mittels der ersten Feder relativ zu dem Signalglied in die distale Richtung bewegbar ist und/oder das Signalglied für die Signalauslösung noch nicht gelöst ist.

In alternativen Ausführungen kann der Ausschütthub drei Phasen, insbesondere einen weiteren Teilhub umfassen. Während des ersten Teilhubs bewegt sich das Vortriebsglied axial relativ zu dem Signalglied. Während des ersten Teilhubs wird das Signalglied nicht geladen. Während des zweiten Teilhubs ist das erste Eingriffsglied des Signalglieds in dem axialfesten Eingriff mit dem Vortriebsglied und das zweite Eingriffsglied des Signalglieds aus dem axialfesten Eingriff mit der Nadelschutzhülse oder dem Schaltmodul, insbesondere der Sperrhülse. Während des zweiten Teilhubs wird das Signalglied geladen. Während des dritten Teilhubs des Ausschütthubs ist das zweite Eingriffsglied in dem axialfesten Eingriff mit der Nadelschutzhülse oder dem Schaltmodul, insbesondere der Sperrhülse, wobei das erste Eingriffsglied aus dem Eingriff mit dem Vortriebsglied ist, wodurch vorteilhaft bewirkt wird, dass das Vortriebsglied mittels der ersten Feder relativ zu dem Signalglied in die distale Richtung bewegbar ist und/oder das Signalglied für die Signalauslösung noch nicht gelöst ist.

Allgemein bevorzugt ist, dass das Vortriebsglied mittels der ersten Feder relativ zu dem Signalglied, insbesondere um den zweiten Teilhub, in die distale Richtung bewegbar ist, wenn das erste Eingriffsglied des Signalglieds aus dem Eingriff mit dem Vortriebsglied und das zweite Eingriffsglied des Signalglieds in dem Eingriff mit der Nadelschutzhülse oder dem Schaltmodul ist.

In bevorzugten Ausführungen können das zweite Eingriffsglied des Signalglieds und die Ausnehmung für das zweite Eingriffsglied im Auslieferungszustand des Autoinjektors entlang der Längsachse in etwa mit dem Abstand zueinander angeordnet sein, der in etwa die Summe aus dem Betätigungshub der Nadelschutzhülse und dem ersten Teilhub des Vortriebsglieds, der in etwa dem Hub des Signalglieds von dem Signalanschlag weg entspricht, beträgt.

Bevorzugt kann das Vortriebsglied das zweite Eingriffsglied des Signalglieds daran hindern, aus dem axialfesten Eingriff mit der Nadelschutzhülse oder dem Schaltmodul auszurücken, wenn sich das Vortriebsglied relativ zu dem Signalglied in die distale Richtung bewegt, insbesondere während des zweiten Teilhubs des Vortriebsglieds. Das Vortriebsglied erlaubt dem zweiten Eingriffsglied am Ende des Ausschütthubs bzw. des zweiten Teilhubs, dass es aus dem Eingriff mit der Nadelschutzhülse oder dem Schaltmodul ausrückt. Wenn das zweite Eingriffsglied am Ende des zweiten Teilhubs aus dem Eingriff mit der Nadelschutzhülse oder dem Schaltmodul ausgerückt ist, wird das Signalglied von der zweiten Feder entgegen die Ausschüttrichtung beschleunigt und schlägt an dem Signalanschlag an. Vorzugsweise wird das zweite Eingriffsglied in dem Eingriff mit der Nadelschutzhülse oder dem Schaltmodul durch den Außenumfang des Vortriebsglieds gehalten, an dem das erste Eingriffsglied anliegt.

In bevorzugten Ausführungen kann der Autoinjektor ein Halteelement aufweisen, an dem sich z. B. ein Ende der ersten Feder, insbesondere das proximale Ende der ersten Feder, abstützt. Alternativ kann sich die Feder mit ihrem proximalen Ende an dem Gehäuse oder einem gehäusefesten Element abstützen. Das Halteelement selbst kann gehäusefest oder in Bezug auf das Gehäuse verschiebbar angeordnet sein. Das Halteelement kann ein erstes Eingriffselement aufweisen, welches vor dem Auslösen der Produktausschüttung in das Vortriebsglied eingreift, wodurch das Vortriebsglied daran gehindert wird, sich relativ zu dem Halteelement und/oder dem Gehäuse in die Ausschüttrichtung zu bewegen. Der Eingriff des ersten Eingriffselements in das Vortriebsglied ist für die Produktausschüttung lösbar. Wenn der Eingriff gelöst ist, ist das Vortriebsglied für die Bewegung in die Ausschüttrichtung freigegeben. Die erste Feder kann das Vortriebsglied relativ zu dem Halteelement und/oder dem Gehäuse um den Ausschütthub in die Ausschüttrichtung verschieben.

Das Vortriebsglied kann eine Ausnehmung für das erste Eingriffselement des Halteelements aufweisen, wobei diese Kopplung zwischen dem Vortriebsglied und dem Halteelement gelöst ist, wenn das Halteelement, insbesondere das erste Eingriffselement, aus dem Eingriff mit dem Vortriebsglied, insbesondere der Ausnehmung des Vortriebsglieds ausgerückt ist. Insbesondere kann das erste Eingriffselement dadurch aus dem Eingriff mit dem Vortriebsglied gelöst werden, dass die Nadelschutzhülse aus der Ausgangsposition um den Betätigungshub in die betätigte Position verschoben wird. Z. B. kann das erste Eingriffselement von der Nadelschutzhülse oder dem Schaltmodul, insbesondere der Sperrhülse in den axialfesten Eingriff mit dem Vortriebsglied gehalten werden, wenn die Nadelschutzhülse nicht in ihrer betätigten Position oder in ihrer Ausgangsposition ist. Z. B. kann ein Innenumfang der Nadelschutzhülse oder des Schaltmoduls, insbesondere der Sperrhülse, das erste Eingriffselement in dem Eingriff mit dem Vortriebsglied halten, wobei z. B. ein zweites Eingriffselement, welches weiter unten beschrieben wird, an dem Innenumfang anliegen kann.

Durch das Verschieben der Nadelschutzhülse in ihre betätigte Position kann die Nadelschutzhülse oder das Schaltmodul, insbesondere die Sperrhülse, dem ersten Eingriffselement des Halteelements erlauben, insbesondere mit einer Bewegung quer zu der Längsachse des Autoinjektors aus dem Eingriff mit dem Vortriebsglied auszurücken. Beispielsweise kann eine Ausnehmung, insbesondere für das zweite Eingriffselement des Halteelements, welche an der Nadelschutzhülse oder dem Schaltmodul, insbesondere der Sperrhülse, gebildet ist, bezogen auf die Längsachse auf der gleichen Position angeordnet sein, wie das erste und/oder zweite Eingriffselement, so dass das erste Eingriffselement aus dem Eingriff mit dem Vortriebsglied ausrücken kann. Beispielsweise kann das Vortriebsglied das erste Eingriffselement aus dem Eingriff mit dem Vortriebsglied drücken, wenn die Nadelschutzhülse in ihrer betätigten Position ist.

Das erste Eingriffselement des Halteelements kann z. B. radial zu der Längsachse hinweisen und/oder an einem federnden Arm des Halteelements angeordnet sein.

Das Halteelement kann - wie erwähnt - ein zweites Eingriffselement aufweisen, welches durch die Ausrückbewegung des ersten Eingriffselements aus dem Vortriebsglied in einen axialfesten Eingriff mit der Nadelschutzhülse oder dem Schaltmodul, insbesondere der Sperrhülse bewegbar ist. Das zweite Eingriffselement kann z. B. an dem Arm, an dem das erste Eingriffselement angeordnet ist, angeordnet sein und/oder z. B. radial von der Längsachse weg weisen. Das erste Eingriffselement und das zweite Eingriffselement können so aufeinander abgestimmt sein, dass das zweite Eingriffselement bereits axialfest in seine Ausnehmung, welche von der Nadelschutzhülse oder dem Schaltmodul, insbesondere der Sperrhülse, gebildet wird, eingreift, wenn das erste Eingriffselement noch nicht vollständig aus dem Eingriff mit dem Vortriebsglied gelöst ist. Hierdurch wird vorteilhaft erreicht, dass zuerst die axialfeste Verbindung zwischen dem Halteelement und der Nadelschutzhülse oder dem Schaltmodul hergestellt ist, bevor die axialfeste Verbindung zwischen dem Halteelement und dem Vortriebsglied gelöst und damit ein erneutes oder weiteres Zurückschieben der Nadelschutzhülse gesperrt wird.

Insbesondere wenn das zweite Eingriffselement des Halteelements in seiner Ausnehmung ist, kann sich das Vortriebsglied relativ zu dem Halteelement in distale Richtung bewegen, insbesondere aufgrund der in der vorgespannten Feder gespeicherten Energie. Das Vortriebsglied kann das zweite Eingriffselement daran hindern, aus dem axialfesten Eingriff mit der Nadelschutzhülse oder dem Schaltmodul, insbesondere der Sperrhülse auszurücken, wenn sich das Vortriebsglied relativ zu dem Signalglied in die distale Richtung bewegt. Dies gilt vorzugsweise auch am Ende des Ausschütthubs, insbesondere auch dann, wenn das zweite Eingriffsglied des Signalglieds aus seiner Ausnehmung gelöst ist, um von der zweiten Feder entgegen die Ausschüttrichtung beschleunigt zu werden.

Insbesondere in Ausführungen, in denen die Ausnehmung für das zweite Eingriffselement des Halteelements von der Nadelschutzhülse oder der Schalthülse gebildet wird, ist es bevorzugt, dass das zweite Eingriffselement am Ende des Ausschütthubs aus seiner Ausnehmung ausrückt, um die Nadelschutzhülse nach der Verabreichung des Produkts aus der betätigten Position in die Nadelschutzposition bewegen zu können. Das Vortriebsglied kann hierzu eine Ausnehmung aufweisen, in welche das erste Eingriffselement einrücken kann, wobei das zweite Eingriffselement gleichzeitig aus seiner Ausnehmung ausrückt, um insbesondere die Bewegung der Nadelschutzhülse in distale Richtung freizugeben.

In Ausführungsformen, mit einem Schaltmodul, welches eine Schalthülse und eine Sperrhülse aufweist, ist es bevorzugt, dass das zweite Eingriffselement auch am Ende des Ausschütthubs so verbleibt, dass das zweite Eingriffselement die Sperrhülse daran hindert, relativ zu dem Gehäuse und/oder dem zweiten Eingriffselement in die distale Richtung bewegt zu werden, wobei die Schalthülse und/oder die Nadelschutzhülse relativ zu der Sperrhülse, insbesondere aufgrund der in der zweiten Feder gespeicherten Energie in die distale Richtung verschiebbar sind, wodurch die Nadelschutzhülse insbesondere in ihre Nadelschutzposition bewegt wird. Wie bereits beschrieben und nur der Vollständigkeit halber angemerkt, kann das Verriegelungsglied zwischen der Sperrhülse und der Schalthülse in einen Eingriff gelangen, der verhindert, dass die Schalthülse relativ zu der Sperrhülse in die proximale Richtung verschiebbar ist. Vorzugsweise wird eine Bewegung der Sperrhülse in die proximale Richtung dadurch verhindert, dass die Sperrhülse entweder an dem Gehäuse oder einem gehäusefesten Element, wie z. B. an einem Mechanikhalter, oder dem Signalglied anstößt.

Ein weiterer Aspekt der Erfindung betrifft die Ausgestaltung eines Produktbehälterhalters, insbesondere eines Spitzenhalters für einen Autoinjektor, insbesondere für einen Autoinjektor, bei dem der Produktbehälter in Bezug auf das Gehäuse unverschiebbar ist bzw. für einen Autoinjektor der oben beschriebenen Art.

Die Erfindung geht von einem Spritzenmodul aus, welches insbesondere zur Verwendung in einem Autoinjektor vorgesehen ist. Insbesondere kann ein Autoinjektor vorgesehen sein, der ein solches Spritzenmodul aufweist. Das Spritzenmodul umfasst eine Spritze und einen Spritzenhalter. Die Spritze weist einen Spritzenkörper, einen Kolben und eine Nadel auf, wobei die Nadel z. B. unlösbar an einem Nadelhalteabschnitt der Spritze befestigt ist und der Kolben in einem zylindrischen Abschnitt des Spritzenkörpers verschiebbar angeordnet ist, wobei der Spritzenkörper einen sich verjüngenden Abschnitt oder Bereich aufweist, der zwischen dem Nadelhalteabschnitt und dem zylindrischen Abschnitt angeordnet ist. Die Spritze weist ferner eine Nadelschutzkappe auf, welche z. B. ein sogenanntes soft needle shield oder vorzugsweise ein rigid needle shield sein kann. Ein soft needle shield ist vorzugsweise aus einem gummielastischen Kunststoff gebildet, wobei ein rigid needle shield aus einer Hülse aus Hartkunststoff gebildet ist, in der eine Hülse aus einem gummielastischen Kunststoff angeordnet ist. Die Hülse aus gummielastischem Kunststoff und die Hülse aus Hartkunststoff bilden zusammen das rigid needle shield. Die Nadelschutzkappe, welche die Nadel abdeckt und an dem sich insbesondere konisch in Richtung Nadelspitze erstreckenden Nadelhalteabschnitt befestigt ist, hält die Nadel vorzugsweise geschützt vor Schmutz und steril. Zwischen dem sich verjüngenden Abschnitt und der Nadelschutzkappe, insbesondere der Hülse aus Hartkunststoff, ist eine Lücke gebildet.

Der Spritzenhalter weist mindestens ein Eingriffsglied, insbesondere eine Schulter, an welcher sich der verjüngende Abschnitt der Spritze in die distale Richtung abstützt und welche in die Lücke zwischen der Nadelschutzkappe und dem sich verjüngenden Abschnitt eingreift, auf. Vorteilhaft wird durch das Anliegen des sich verjüngenden Abschnitts an der mindestens einen Schulter verhindert, dass sich die Spritze relativ zu dem Spritzenhalter in die distale Richtung bewegen kann.

Es ist bevorzugt, dass zwischen der Schulter und der Nadelschutzkappe ein Spalt ist oder besteht, so dass die Nadelschutzkappe von der Schulter unbelastet bleibt. Hierdurch wird vorteilhaft verhindert, dass die Sterilität der Nadel durch ein unbeabsichtigtes Verschieben der Nadelschutzkappe durch die Schulter beeinträchtigt wird.

In bevorzugten Ausführungen kann der Spritzenkörper an seinem proximalen Ende einen Fingerflansch aufweisen, wobei zwischen dem Fingerflansch und dem Spritzenkörper ein Spalt gebildet ist, wenn der sich verjüngende Abschnitt an der Schulter anliegt, wodurch der Fingerflansch im Wesentlichen unbelastet bleibt. Hierdurch wird vorteilhaft verhindert, dass der Fingerflansch überbelastet wird und den Spritzenkörper zerbricht.

Es ist ferner bevorzugt, dass der Spritzenhalter mindestens ein Halteglied, insbesondere eine nach außen gerichtete Abragung aufweist, mit dem der Spritzenhalter axialfest mit einem Gehäuse des Autoinjektors verbindbar oder verbunden ist, insbesondere verschnappt oder verschnappbar ist.

Insbesondere kann der Spritzenhalter mindestens einen Nocken aufweisen, der federnd, insbesondere an einem Arm angeordnet ist und z. B. distal des Halteglieds angeordnet ist. Der mindestens eine Nocken kann eine Nadelschutzhülse an der Bewegung aus ihrer Ausgangsposition in ihre betätigte Position so hemmen oder hindern, dass beim Überschreiten einer auf die Nadelschutzhülse entlang der Längsachse L des Autoinjektors ausgeübten Grenzkraft der mindestens eine Nocken aus dem Eingriff mit der Nadelschutzhülse gedrückt wird, wodurch die Nadelschutzhülse schlagartig in ihre betätigte Position relativ zu dem Gehäuse verschiebbar ist.

Das Gehäuse des Autoinjektors kann z. B. einen Halteabschnitt aufweisen, der an dem Spritzenhalter, insbesondere an einer Außenfläche oder einem Außenumfang des Spritzenhalters anliegt und das mindestens eine Eingriffsglied des Spritzenhalters daran hindert, sich quer zur Längsachse von der Längsachse weg zu bewegen. Insbesondere kann der Halteabschnitt zylinderförmig sein und das mindestens eine Eingriffsglied, vorzugsweise zwei oder drei oder vier Eingriffsglieder umgeben, so dass das mindestens eine Eingriffsglied innerhalb des Halteabschnitts angeordnet ist. Für die Montage bzw. das Einlegen der Spritze in den Spritzenhalter befindet sich der Spritzenhalter außerhalb des Eingriffs mit dem Halteabschnitt des Gehäuses. Wenn die Spritze vollständig in den Spritzenhalter eingelegt ist, insbesondere das mindestens eine Eingriffsglied in die Lücke zwischen dem sich verjüngenden Abschnitt und der Nadelschutzkappe eingreift, wird das Spritzenmodul bzw. der Spritzenhalter in den Eingriff mit dem Halteabschnitt gebracht, so dass verhindert wird, dass sich das mindestens eine Eingriffsglied aus dem Eingriff mit dem verjüngenden Abschnitt quer zur Längsachse, insbesondere von der Längsachse weg oder nach außen, bewegt.

In einer ersten Variante kann das mindestens eine Eingriffsglied federnd, insbesondere an einem Arm an dem Spritzenhalter gebildet sein, wobei die Spritze über das proximale Ende mit der Nadel voraus in den Spritzenhalter, der vorzugsweise hülsenförmig ist, eingeschoben wird, wobei die Nadelschutzkappe das mindestens eine Eingriffsglied quer zur Längsachse nach außen, d. h. von der Längsachse weg auslenkt, wobei, wenn die Nadelschutzkappe vollständig an dem mindestens einen Eingriffsglied vorbei bewegt wurde, das mindestens eine Eingriffsglied in die Lücke zwischen dem sich verjüngenden Bereich und der Nadelschutzkappe einschnappt. Anschließend wird der Spritzenhalter mit der Spritze in den Eingriff mit dem Halteabschnitt des Gehäuses des Autoinjektors verschoben, wodurch das mindestens eine Eingriffsglied in den Eingriff mit der Lücke zwischen der Nadelschutzkappe und dem sich verjüngenden Abschnitt gehalten wird und aus diesem Eingriff nicht mehr herausfedern kann.

Das Gehäuse umfasst einen Halteabschnitt. Der Halteabschnitt des Gehäuses kann hülsenförmig ausgebildet sein und im Innern des Gehäuses angeordnet sein. Zwischen dem hülsenförmigen Halteabschnitt des Gehäuses und dem hülsenförmigen Gehäuse kann ein Anschlag zur Begrenzung der axialen Bewegung der Nadelschutzhülse in die proximale Richtung vorgesehen sein. Vorzugsweise ragt der Halteabschnitt des Gehäuses in distaler Richtung über das distale Ende des Gehäuses über. Ferner kann der Halteabschnitt eine oder mehrere Rillen aufweisen, welche in Verbindung mit an der Nadelschutzhülse vorgesehener Schiene oder Schienen eine Verdrehsicherung zwischen dem Gehäuse und der Nadelschutzhülse bilden. Die Schiene der Nadelschutzhülse ist vorzugsweise an einer Mantelinnenfläche der Nadelschutzhülse vorgesehen. Die Rille des Halteabschnitts ist vorzugsweise an einer Mantelaussenfläche des Halteabschnitts angeordnet. Die Nadelschutzhülse kann vorzugsweise drehfest zwischen dem Halteabschnitt des Gehäuses und dem Gehäuse axial bewegt werden. In alternativen Ausführungsformen kann die eine oder die mehreren Rillen an der Nadelschutzhülse und die eine oder die mehreren Schienen an dem Halteabschnitt des Gehäuses vorgesehen sein, um eine Versicherung zwischen dem Gehäuse und der Nadelschutzhülse zu bilden. Die Rille der Nadelschutzhülse ist vorzugsweise an einer Mantelinnenfläche der Nadelschutzhülse vorgesehen. Die Schiene des Halteabschnitts ist vorzugsweise an einer Mantelaussenfläche des Halteabschnitts angeordnet.

Die Erfindung und der weitere Aspekt zur Erfindung wurden anhand mehrerer bevorzugter Ausführungen beschrieben. Im Folgenden werden besonders bevorzugte Ausführungen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1a-1b: Explosionsdarstellungen eines Autoinjektors gemäß einer besonders bevorzugten Ausführungsform,
- Figuren 2a-2c: der Autoinjektor aus Figur 1 in einem Auslieferungszustand, wobei die Figuren 2a bis 2c durch die Längsachse der Vorrichtung verlaufende Schnittansichten sind, wobei die Schnittansichten um die Längsachse winkelversetzt sind,
- Figuren 3a-3c: die Vorrichtung und die Ansichten aus den Figuren 2a-2c, wobei sich eine Nadelschutzhülse in ihrer betätigten Position befindet,
- Figuren 4a-4c: die Vorrichtung und die Ansichten aus den Figuren 2a-2c, wobei das Vortriebsglied am Ende eines ersten Teilhubs seines Ausschütthubs gezeigt wird,
- Figuren 5a-5c: die Vorrichtung und die Ansichten aus den Figuren 2a-2c, wobei ein Vortriebsglied am Ende seines Ausschütthubs gezeigt wird,
- Figuren 6a-6c: die Vorrichtung und die Ansichten aus den Figuren 2a-2c, wobei ein Signal, welches das Ende der Produktausschüttung signalisiert, erzeugt wird,
- Figuren 7a-7c: die Vorrichtung und die Ansichten aus den Figuren 2a-2c, wobei die Nadelschutzhülse in ihrer Nadelschutzposition ist,
- Figur 8: perspektivische Ansichten und Längsschnitte des Vortriebsglieds
- Figur 9: eine alternative Ausführungsform des Vortriebsglieds gemäss Figur 8, wobei die Rippe 7c' eine andere Länge aufweist.

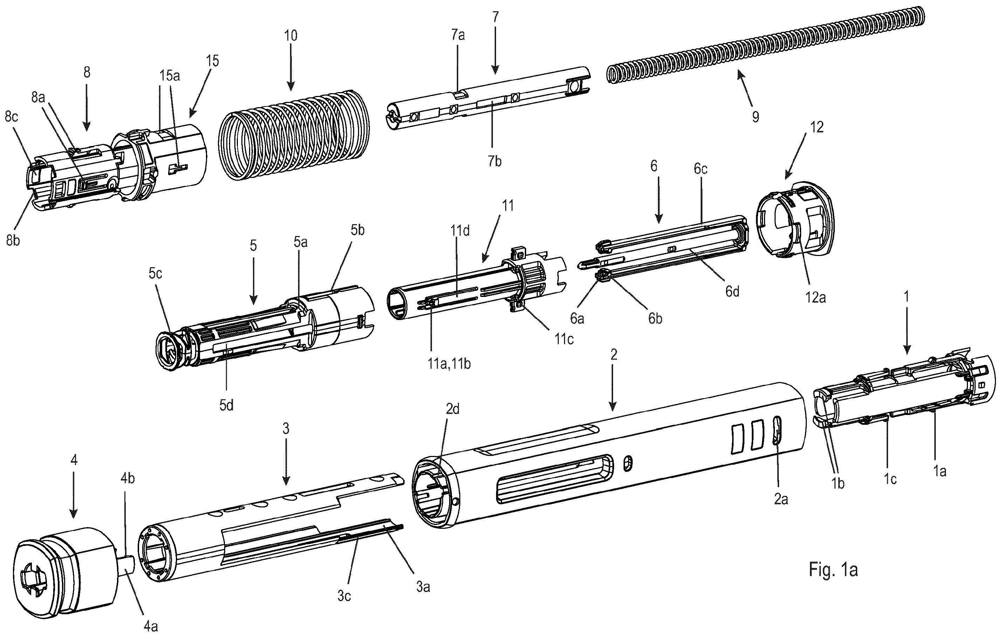

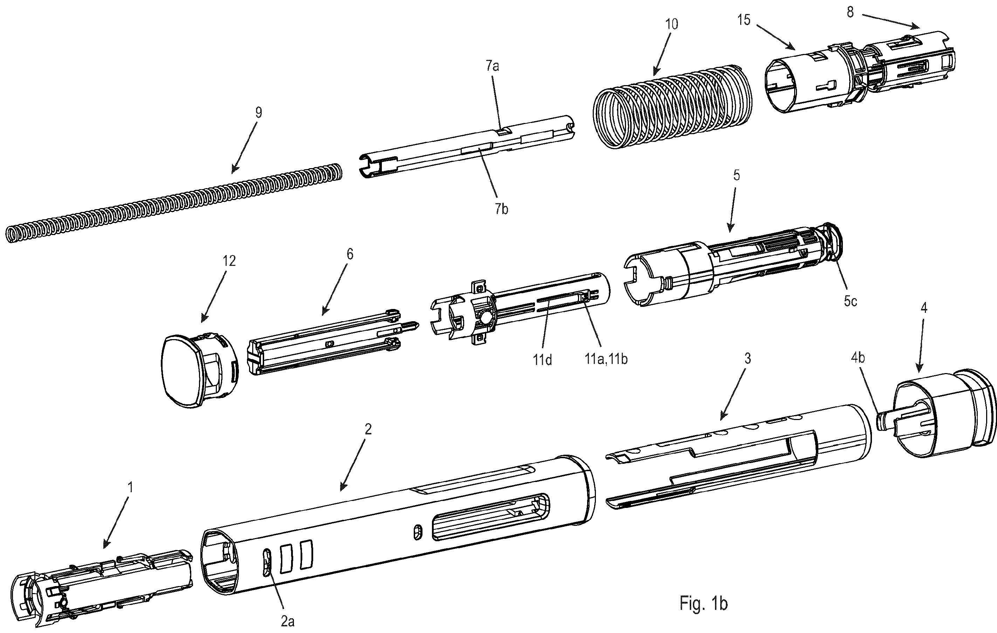

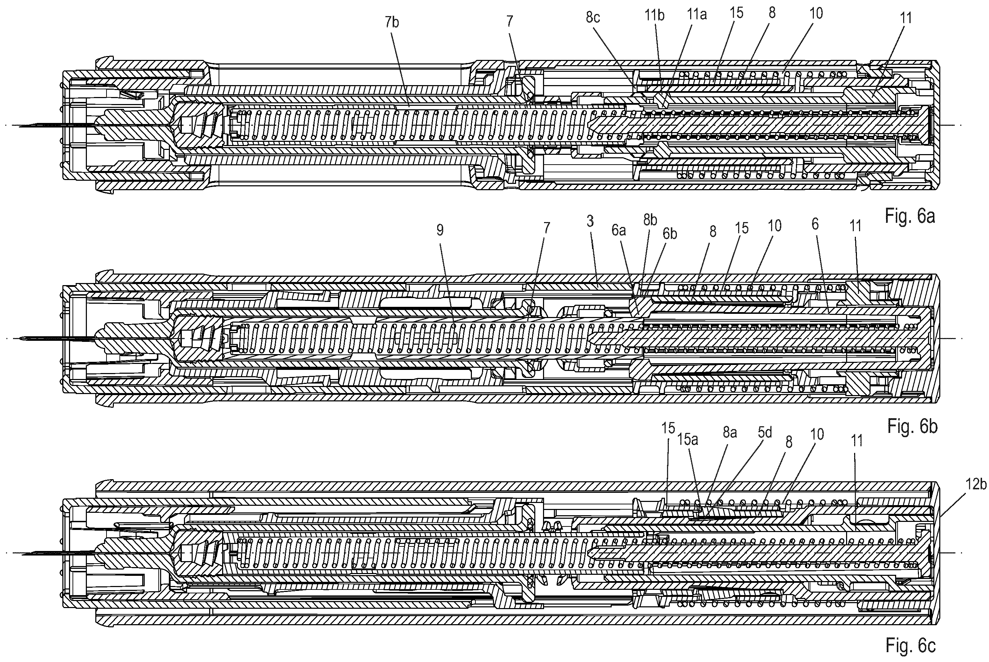

Bezugnehmend auf die Figuren 1-8 werden nun die strukturellen Merkmale und die Funktion des bevorzugten Autoinjektors beschrieben.

Der Autoinjektor weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

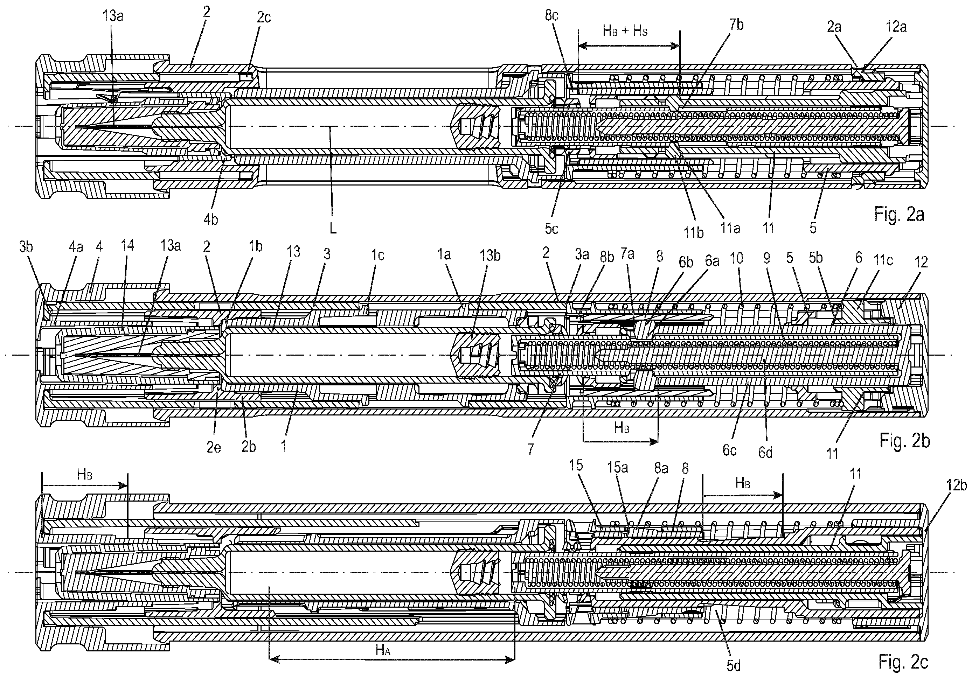

An dem distalen Ende des Autoinjektors ist in seinem Auslieferungszustand (Figuren 2a-2c) eine Abziehkappe 4 angeordnet, die vor der Verwendung des Autoinjektors abgezogen, oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin das Produkt durch die hohle Injektionsnadel 13a aus dem Produktbehälter 13 ausschüttet. Der Spritzenkörper weist an seinem proximalen Ende einen sogenannten Fingerflansch auf, der radial nach außen über den Außenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelschutzkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird. Alternativ kann der Schnapper 4b in eine Mantelfläche des rigid needle shield 14 oder in eine Mantelfläche des soft needle shield einhaken.

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 2a-2c gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag 2c bilden, an dem die Nadelschutzhülse 3 in der betätigten Position anliegt.

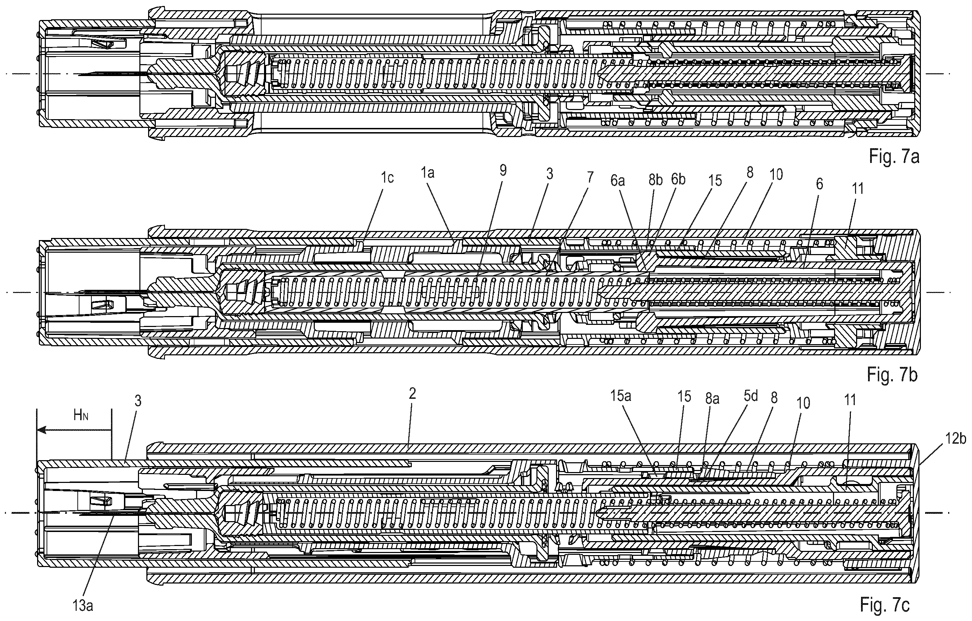

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 7a-7c) verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spritzenhalter 1 weist eine Abragung 1a, die radial nach außen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2c) und/oder in der Nadelschutzposition der Nadelschutzhülse 3 (Figuren 7a-7c) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 in die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen zylinderförmigen Halteabschnitt oder Zylinderabschnitt 2b auf, der insbesondere das distale Ende des Spritzenhalters 1 insbesondere zylinderförmig umgibt, und daran anliegt, wodurch die mindestens eine Schulter 1b in dem Eingriff mit dem verjüngenden Bereich des Spritzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhalter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten. Ferner kann der zylinderförmige Halteabschnitt 2b Rillen 2d aufweisen, welche in Verbindung mit Schienen 3c welche an der Innenseite der Nadelschutzhülse 3 angebracht sind eine Verdrehsicherung für die Nadelschutzhülse 3 bilden.

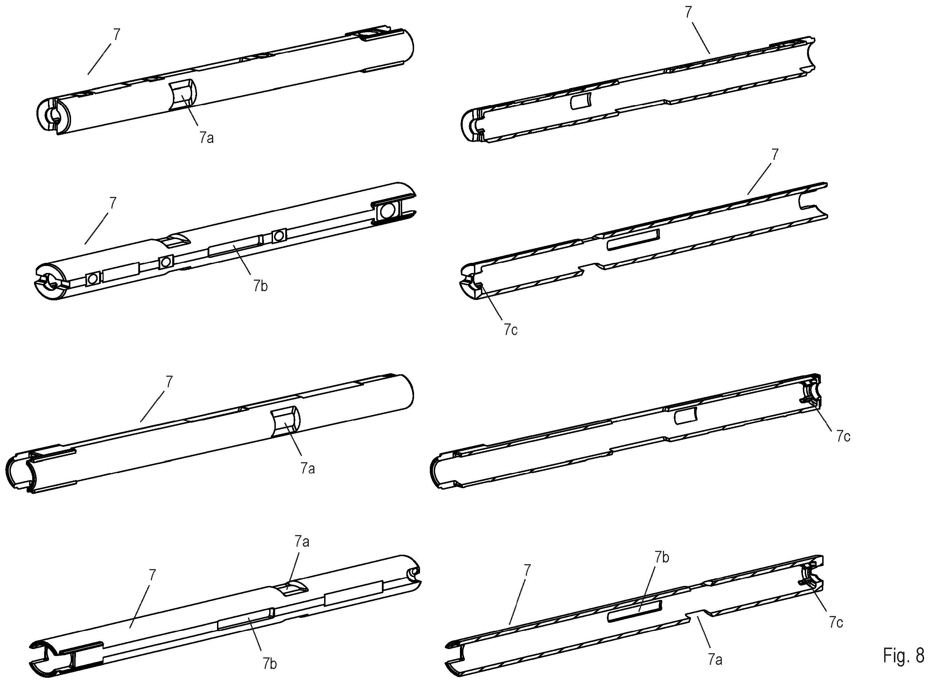

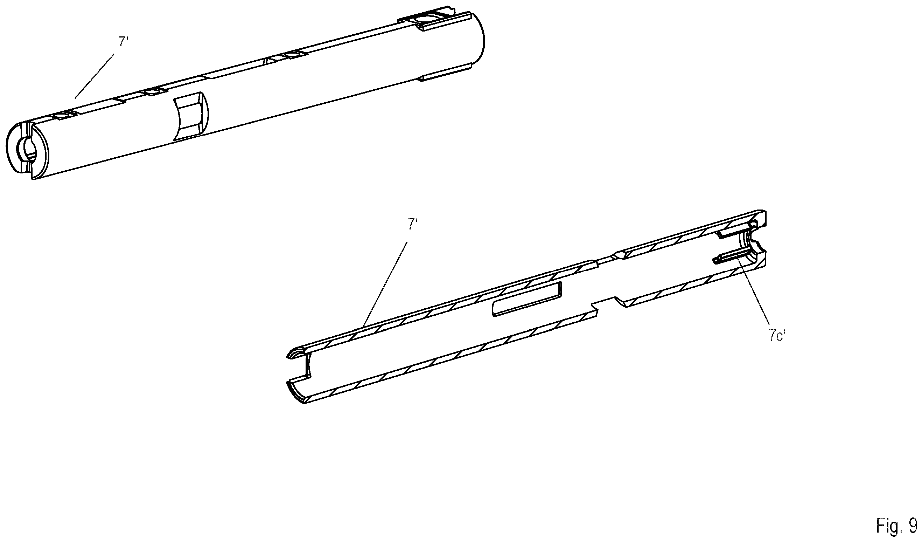

Der Autoinjektor weist ferner ein hülsenförmiges Vortriebsglied 7; 7' auf (Fig.8; Fig.9), welches an seinem distalen Ende nach innen und in Längsrichtung ragende Rippen 7c, 7c' bildet, an der sich eine erste Feder 9, die auch als Ausschüttfeder bezeichnet werden kann, abstützt. Die erste Feder 9 ist innerhalb des hülsenförmigen Vortriebsglieds 7; 7' angeordnet. Die Länge der Rippen 7c; 7c' ist so ausgestaltet, dass der Einbauraum für die erste Feder 9, die eine als Druckfeder wirkende Wendelfeder ist, verkleinert wird und so die Feder 9 im Ausgangs- oder Auslieferungszustand des Autoinjektors mit so viel Energie vorgespannt ist, dass sie das in dem Produktbehälter 13 enthaltene Produkt, insbesondere vollständig durch Verschieben des Vortriebsglieds 7; 7' um einen Ausschütthub H_{A} aus dem Produktbehälter 13 ausschütten kann. Zusätzlich bilden die Rippen 7c, 7c' eine Verstärkung des Bodens, des Vortriebsglieds 7; 7', so dass der distale Bereich des Vortriebsglieds 7; 7', bedingt durch die hohen Kräfte der Ausschüttfeder 9 nicht durchbricht. Die Länge der Rippen 7c; 7c' kann in verschiedenen Autoinjektoren unterschiedlich sein, wobei die entsprechende Länge der Rippen des Vortriebsglieds 7; 7' zur Einstellung der Federspannung der in den verschiedenen Autoinjektoren identischen ersten Feder dienen kann. Z.B. können länger ausgebildete Rippen eine höhere Federvorspannung erzeugen, zur Ausschüttung einer höherviskosen Flüssigkeit. Es können also zwei bis auf die Rippenlänge identische Autoinjektoren gebildet werden mit unterschiedlicher Federvorspannung. Es kann auch nur eine Rippe 7c; 7c' im entsprechenden Vortriebsglied 7; 7' vorgesehen sein. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs H_{A} an den Kolben 13b anschlägt und diesen in die Ausschüttrichtung mitnimmt.

Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten. Das Halteelement 6 weist einen Führungsstift 6d auf, der durch das proximale Ende der ersten Feder 9 in die Seele der Feder 9 eingefügt ist. Der Führungsstift 6d verhindert ein seitliches Ausknicken der ersten Feder 9 während und am Ende des Ausschütthubs H_{A} des Vortriebsglieds 7.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der ersten Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, innerhalb der die erste Feder 9 angeordnet ist und die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an einem Signalglied 11, insbesondere an einer Abragung 11c, die axial verschiebbar und verdrehfest in das Gehäuse 2 eingreift und die durch eine schlitzförmige Nut 5b des Mechanikhalters 5 hindurchgreift, ab. Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Das insbesondere hülsenförmige Signalglied 11 ist im Auslieferungszustand oder vor der Auslösung der Produktausschüttung in einem axialfesten Eingriff mit dem Vortriebsglied 7. Das Signalglied 11 weist ein erstes Eingriffsglied 11a, welches in eine Ausnehmung 7b des Vortriebsglieds 7 eingreift, und ein zweites Eingriffsglied 11b auf. Das erste Eingriffsglied 11a und das zweite Eingriffsglied 11b sind an dem Ende eines Arms 11d federnd angeordnet. Das Signalglied 11 weist zwei solcher Arme 11d mit einem ersten Eingriffsglied 11a und einem zweiten Eingriffsglied 11b auf. Das erste Eingriffsglied 11a weist radial zu der Längsachse L hin, wobei das zweite Eingriffsglied 11b radial von der Längsachse L weg weist. Im Auslieferungszustand wird das erste Eingriffsglied 11a von dem Innenumfang der Sperrhülse 8 in dem axialfesten Eingriff mit dem Vortriebsglied 7 gehalten. In alternativen Ausführungen kann die Ausnehmung 7b derart ausgebildet sein, insbesondere eine in Längsrichtung erstreckende Ausnehmung 7b sein, dass während eines ersten Teilhubs des Ausschütthubs eine axiale Relativbewegung zwischen dem Vortriebsglied 7 und dem Signalglied 11 stattfindet und während eines zweiten Teilhubs des Ausschütthubs das erste Eingriffsglied 11a in dem axialfesten Eingriff zumindest in distaler Richtung mit dem Vortriebsglied 7 gehalten wird. Das zweite Eingriffsglied 11b liegt an dem Innenumfang der Schalthülse 8 an. Die Verschlusskappe 12 weist einen Signalanschlag 12b auf, an den das Signalglied 11 zur Erzeugung eines Signals anschlagen kann und vorzugsweise an dem das Signalglied 11 im Auslieferungszustand der Vorrichtung anliegt.

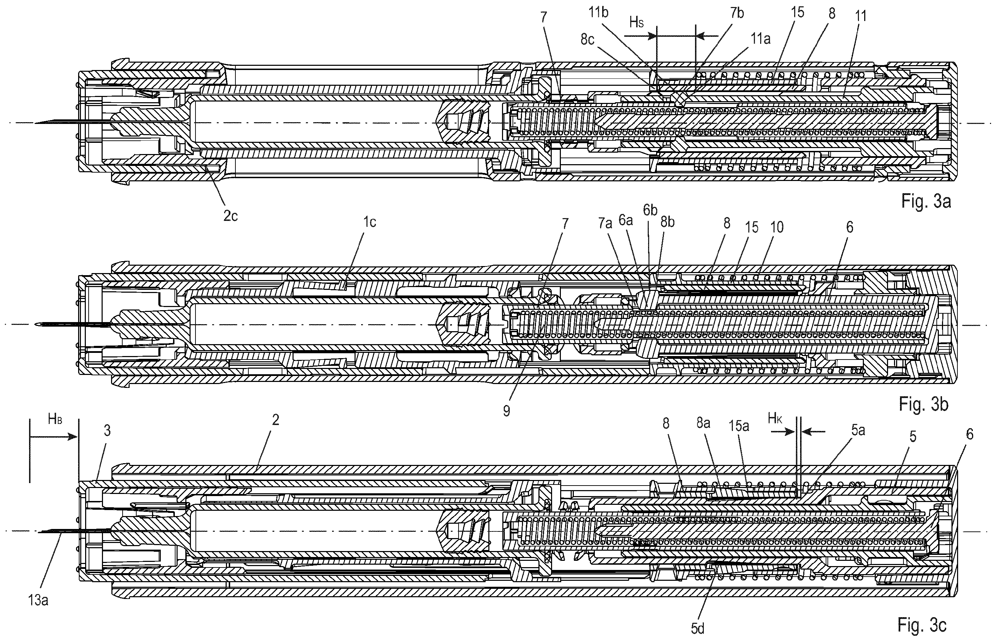

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine erste Ausnehmung 8b auf, die durch Verschieben der Sperrhülse 8 um den Betätigungshub H_{B} entlang der Längsachse L auf die Position des zweiten Eingriffselements 6b gebracht wird, wie in den Figuren 3a-3c dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren erster Ausnehmung 8b bewegt wird. Hierdurch wird das Vortriebsglied 7 für die Bewegung um den Ausschütthub H_{A} in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der ersten Feder 9 in die proximale Richtung bewegt werden, wobei das Halteelement 6 über den Eingriff des zweiten Eingriffselements 6b in die Ausnehmung 8b die Sperrhülse 8 um einen Startsignalhub H_{K} (Figur 3c) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist.

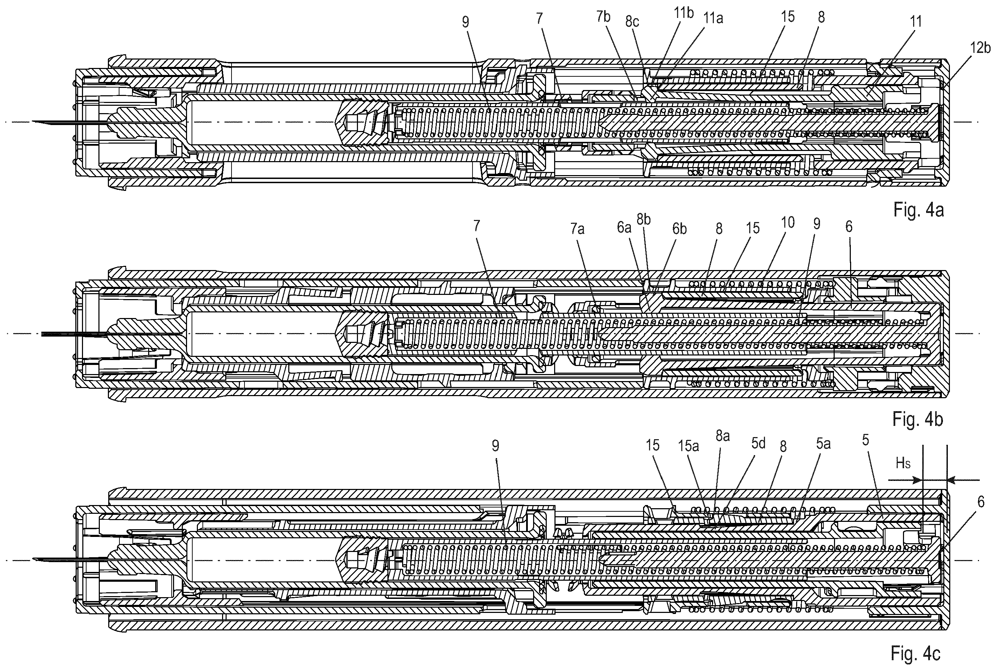

Da das Signalglied 11 noch axialfest mit dem Vortriebsglied 7 verbunden ist, wird es um einen ersten Teilhub H_{S} des Ausschütthubs H_{A} in die Ausschüttrichtung mitgenommen, wobei das Signalglied 11 in etwa um den ersten Teilhub H_{S} von dem Signalanschlag 12b weg bewegt wird, wie am besten aus Figur 4c erkennbar ist. Am Ende des ersten Teilhubs H_{S}, während dem das erste und zweite Eingriffsglied 11a, 11b relativ zu der Sperrhülse 8 bewegt werden, wird das erste Eingriffsglied 11a aus dem Eingriff mit dem Vortriebsglied 7 gedrückt, wobei gleichzeitig das zweite Eingriffsglied 11b in die zweite Ausnehmung 8c der Sperrhülse 8 mit einer Bewegung quer zu der Längsachse L und radial von der Längsachse L weg bewegt wird. Hierdurch wird das Signalglied 11 daran gehindert, sich in die proximale Richtung relativ zu dem Gehäuse 2 oder der Sperrhülse 8 zu bewegen. Das zweite Eingriffsglied 11b wird von dem Außenumfang des Vortriebsglieds 7 in den Eingriff mit der Ausnehmung 8c gehalten (Figur 4a), wenn das Vortriebsglied 7 um seinen zweiten Teilhub des Ausschütthubs H_{A} bewegt wird. Die Außenumfangsfläche des Vortriebsglieds 7 hält das zweite Eingriffselement 6b in dem Eingriff mit der ersten Ausnehmung 8b der Sperrhülse 8, wie am besten aus Figur 4b erkennbar ist. Am Ende des Ausschütthubs H_{A} gibt das Vortriebsglied 7 das zweite Eingriffsglied 11b aus dem Eingriff mit der Sperrhülse 8 frei, wodurch das zweite Eingriffsglied 11b aus dem Eingriff mit der Ausnehmung 8c bewegt wird, insbesondere zu der Längsachse L hin, so dass die zweite Feder 10 das Signalglied 11 entgegen die Ausschüttrichtung, d. h. in die proximale Richtung beschleunigt, so dass beim Auftreffen des Signalglieds 11 auf dem Signalanschlag 12b ein akustisches und/oder taktiles Signal erzeugt wird.

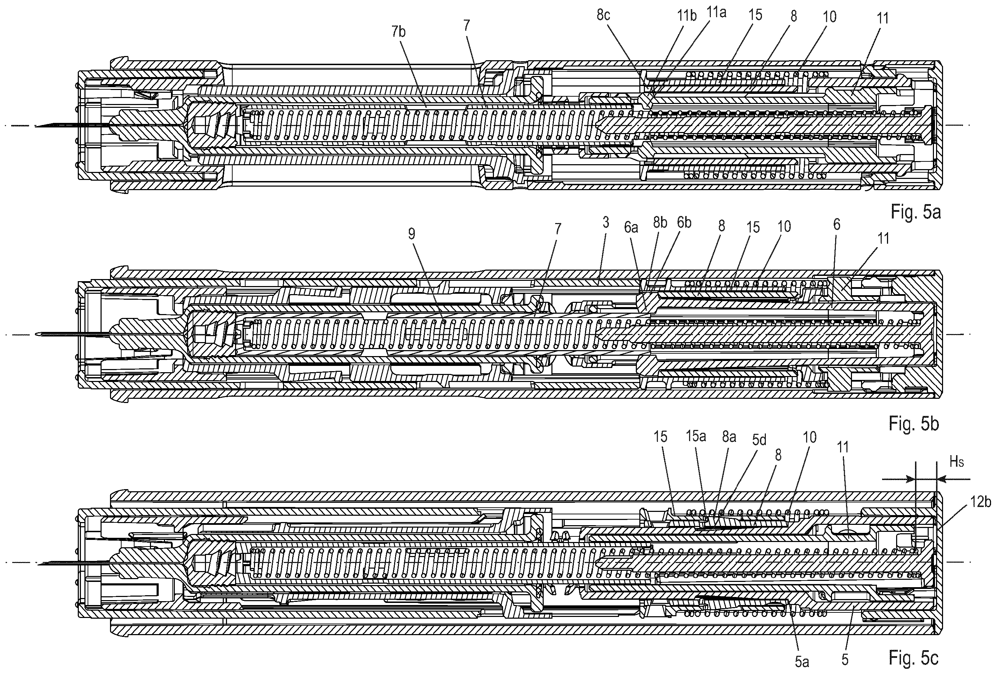

Wie am besten aus Figur 5b erkennbar ist, bleibt der Eingriff des zweiten Eingriffselements 6b in die erste Ausnehmung 8b bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen, wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stößt das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

### Bezugszeichenliste

| | | | |
|---|---|---|---|
| 1 | Spritzenhalter | 8 | Sperrhülse |
| 1a | Abragung/ Halteglied | 8a | Verriegelungsglied |
| 1b | Schulter/ Eingriffsglied | 8b | erste Ausnehmung |
| 1c | Nocken | 8c | zweite Ausnehmung |
| 2 | Gehäuse | | |
| 2a | Ausnehmung | 9 | erste Feder/Ausschüttfeder |
| 2b | Zylinderabschnitt/Halteabschnitt | 10 | zweite Feder/Nadelschutzfeder |
| 2c | Betätigungsanschlag | | |
| 2e | Halteschulter | 11 | Signalglied |
| 2d | Rillen | 11a | erstes Eingriffsglied |
| | | 11b | zweites Eingriffsglied |
| 3 | Nadelschutzhülse | 11c | Abragung |
| 3a | proximales Ende | 11d | Arm |
| 3b | Schnappgeometrie | | |
| 3c | Schienen | 12 | Verschlusskappe |
| | | 12a | Rastglied |
| 4 | Abziehkappe | 12b | Signalanschlag |
| 4a | Schnapphaken | | |
| 4b | Schnapper | 13 | Produktbehälter/Spritze |
| | | 13a | Nadel |
| 5 | Mechanikhalter | 13b | Kolben |
| 5a | Startsignalanschlag | | |
| 5b | Nut | 14 | rigid needle shield/ Nadelschutzkappe |
| 5c | Haltefederabschnitt | | |
| 5d | Vertiefung | 15 | Schalthülse |
| | | 15a | Ausnehmung |
| 6 | Halteelement | | |
| 6a | erstes Eingriffselement | H_{A} | Ausschütthub |
| 6b | zweites Eingriffselement | H_{B} | Betätigungshub |
| 6c | Arm | H_{S} | Signalhub/erster Teilhub |
| 6d | Führungsstift | H_{K} | Startsignalhub |
| | | H_{N} | Nadelschutzhub |
| 7; 7' | Vortriebsglied | H_{M} | Montagehub |
| 7a | erste Ausnehmung | | |
| 7b | zweite Ausnehmung | L | Längsachse |
| 7c; 7c' | Rippen | | |

## Patentansprüche

1. Autoinjektor zur Ausschüttung eines flüssigen Produkts, insbesondere Medikaments, umfassend:
a) ein Gehäuse (2) und einen in dem Gehäuse (2) angeordneten Produktbehälter (13), insbesondere Spritze, der einen verschiebbaren Kolben (13b) aufweist, wobei der Kolben (13b) zur Ausschüttung des in dem Produktbehälter (13) enthaltenen Produkts in eine Ausschüttrichtung verschiebbar ist,
b) ein Vortriebsglied (7; 7'), das während der Produktausschüttung auf den Kolben (13b) wirkt, und eine erste Feder (9), die auf das Vortriebsglied (7; 7') wirkt,
c) eine Nadelschutzhülse (3), welche für die Auslösung der Produktausschüttung aus ihrer Ausgangsposition relativ zu dem Gehäuse (2) und entlang einer Längsachse (L) des Autoinjektors in die proximale Richtung, insbesondere um einen Betätigungshub (H_{B}) verschiebbar ist, wodurch eine zweite Feder (10) gespannt und insbesondere die Produktausschüttung ausgelöst wird, wobei die Nadelschutzhülse (3) auf die zweite Feder (10) wirkt,
**dadurch gekennzeichnet, dass**
d) das Gehäuse (2) einen Halteabschnitt umfasst, wobei der Halteabschnitt des Gehäuses in distaler Richtung über ein distales Ende des Gehäuses ragt, und wobei der Halteabschnitt des Gehäuses (2) eine oder mehrere Rillen (2d) oder alternativ eine oder mehrere Schienen aufweist, welche in Verbindung mit an der Nadelschutzhülse (3) vorgesehener Schiene oder Schienen (3c) oder alternativ in Verbindung mit an der Nadelschutzhülse (3) vorgesehener Rille oder Rillen eine Verdrehsicherung zwischen dem Gehäuse (2) und der Nadelschutzhülse (3) bildet, und
e) das Vortriebsglied (7; 7') an einer Innenseite eine nach innen ragende Rippe (7c; 7c') oder nach innen ragende Rippen (7c; 7c') zur Einstellung einer Federspannung der ersten Feder (9) aufweist, wobei die Länge der Rippe (7c;7c') oder der Rippen (7c; 7c') eine Vorspannung der ersten Feder (9) so bestimmt, dass sie das Produkt aus dem Produktbehälter (13b) durch Verschieben des Vortriebsglieds (9) um einen Ausschütthub (H_{A}) ausschütten kann.

2. Autoinjektor nach Anspruch 1, **dadurch gekennzeichnet, dass** der Halteabschnitt des Gehäuses (2) hülsenförmig ausgebildet ist und in dem Innern des Gehäuses (2) angeordnet ist.

3. Autoinjektor nach Anspruch 1 oder 2, **dadurch gekennzeichnet, dass** die Rille (2d) oder die Schiene des Halteabschnitts des Gehäuses (2) an einer Mantelaussenfläche des Halteabschnitts des Gehäuses (2) angeordnet ist.

4. Autoinjektor nach einem der vorhergehenden Ansprüche, **dadurch gekennzeichnet, dass** die Rille oder die Schiene (3c) der Nadelschutzhülse (3) an einer Mantelinnenfläche der Nadelschutzhülse (3) vorgesehen ist.

5. Autoinjektor nach einem der vorhergehenden Ansprüche, **dadurch gekennzeichnet, dass** zwischen dem hülsenförmigen Halteabschnitt des Gehäuses (2) und dem hülsenförmigen Gehäuse (2) ein Anschlag zur Begrenzung der axialen Bewegung der Nadelschutzhülse (3) in die proximale Richtung vorgesehen ist.

6. Autoinjektor nach einem der vorhergehenden Ansprüche, **gekennzeichnet durch** ein Schaltmodul (8, 15), welches kinematisch oder/und geometrisch zwischen der zweiten Feder (10) und der Nadelschutzhülse (3) angeordnet ist, wobei das Schaltmodul (8, 15) von der Nadelschutzhülse (3) in die proximale Richtung mitgenommen wird, wenn die Nadelschutzhülse (3) aus ihrer Ausgangsposition in die proximale Richtung verschoben wird.

7. Autoinjektor nach Anspruch 6, **dadurch gekennzeichnet, dass** das Schaltmodul (8, 15) eine Schalthülse (15) und Sperrhülse (8) aufweist, wobei die Sperrhülse (8) ein unidirektional wirkendes Verriegelungsglied (8a) aufweist, welches in die Schalthülse (15) eingreift, wobei die Schalthülse (15) bei ihrer Bewegung relativ zu dem Gehäuse (2) in die proximale Richtung die Sperrhülse (8) über das Verriegelungsglied (8a) mitnimmt und bei ihrer Bewegung relativ zu dem Gehäuse (2) in proximale Richtung relativ zu der Sperrhülse (8) in eine weitere Sperrposition verschoben wird, in der das Verriegelungsglied (8a) eine Bewegung der Schalthülse (15) relativ zu der Sperrhülse (8) in die proximale Richtung sperrt.

8. Autoinjektor nach einem der vorhergehenden Ansprüche, **dadurch gekennzeichnet, dass** die Nadelschutzhülse (3) nach der Produktausschüttung, insbesondere wenn das Vortriebsglied (7; 7') um den Ausschütthub (H_{A}) in die distale Richtung verschoben ist, von der zweiten Feder (10) relativ zu dem Gehäuse (2) in die distale Richtung, insbesondere um einen Nadelschutzhub (H_{S}) in eine Nadelschutzposition verschiebbar ist, in der die Nadelschutzhülse (3) distal über die Nadelspitze einer Injektionsnadel (13a) des Produktbehälters (13) steht.

9. Autoinjektor nach Anspruch 8, **gekennzeichnet, durch** ein Verriegelungsglied (12a), welches die Nadelschutzhülse (3) in ihrer Nadelschutzposition relativ zu dem Gehäuse (2) gegen Zurückschieben in die proximale Richtung verriegelt, insbesondere zumindest so verriegelt, dass die Nadelspitze nicht aus dem distalen Ende der Nadelschutzhülse (3) hervortreten kann.

10. Autoinjektor nach einem der vorhergehenden Ansprüche, **gekennzeichnet durch** ein Signalglied (11), einen Signalanschlag (12b) und der zweiten Feder (10), welche auf das Signalglied (11) eine entgegen der Ausschüttrichtung wirkende Federkraft ausübt, wobei das Signalglied (11) mit dem Vortriebsglied (7; 7') axialfest gekoppelt ist, so dass das Signalglied (11) während des Verschiebens des Vortriebsglieds (7; 7') in die Ausschüttrichtung mitgenommen und die zweite Feder (10) gespannt wird, wobei die axialfeste Kopplung zwischen Signalglied (11) und Vortriebsglied (7; 7') lösbar ist und das Signalglied (11) mittels der zweiten Feder (10) entgegen der Ausschüttrichtung und relativ zu dem Vortriebsglied (7; 7') oder/und dem Gehäuse (2) beschleunigbar ist, wobei das von der axialfesten Kopplung mit dem Vortriebsglied (7; 7') entkoppelte, von der zweiten Feder (10) beschleunigte Signalglied (11) an dem Signalanschlag (12b) anschlägt, wodurch ein akustisches und/oder taktiles Signal erzeugt wird.

11. Autoinjektor nach Anspruch 10, **dadurch gekennzeichnet, dass** das Signalglied (11) ein erstes Eingriffsglied (11a) aufweist, welches in das Vortriebsglied (7; 7') lösbar eingreift, wodurch das Vortriebsglied (7; 7') axialfest mit dem Signalglied (11) gekoppelt ist, wobei die axialfeste Kopplung zwischen dem Vortriebsglied (7; 7') und dem Signalglied (11) gelöst ist, wenn das Signalglied (11) aus dem Eingriff mit dem Vortriebsglied (7;7') ist.

12. Autoinjektor nach Anspruch 10 oder 11, **dadurch gekennzeichnet, dass** der Signalanschlag (12b) von dem Gehäuse (2) oder einem zumindest axialfest, vorzugsweise auch drehfest, mit dem Gehäuse (2) verbundenen Element (12), wie z.B. einer Verschlusskappe (12), welche das proximale Ende des Gehäuses (2) verschließt, gebildet ist.

13. Autoinjektor nach einem der Ansprüche 10 bis 12, **dadurch gekennzeichnet, dass** der Signalanschlag (12b) entlang der Längsachse (L) des Autoinjektors, insbesondere des Gehäuses (2), in einer Flucht mit dem Signalglied (11) angeordnet ist.

14. Ein erster und ein zweiter Autoinjektor nach einem der vorhergehenden Ansprüche, **dadurch gekennzeichnet, dass** die Länge der Rippe oder Rippen (7c) des ersten Autoinjektors verschieden ist von der Länge der Rippe oder Rippen (7c') des zweiten Autoinjektors, und dass die Autoinjektoren ansonsten identisch sind.

## Claims

1. Autoinjector for dispensing a liquid product, in particular a drug, comprising:
a) a housing (2) and a product container (13) arranged in the housing (2), in particular a syringe, which has a movable plunger (13b), the plunger (13b) being movable in a dispensing direction in order to dispense the product contained in the product container (13),
b) a propulsion element (7; 7') which acts on the plunger (13b) during product dispensing, and a first spring (9) which acts on the propulsion element (7; 7'),
c) a needle protection sleeve (3) which, in order to trigger the product dispensing, is movable from its starting position relative to the housing (2) and along a longitudinal axis (L) of the autoinjector in the proximal direction, in particular by an actuating stroke (Hs), as a result of which a second spring (10) is tensioned and in particular the product dispensing is triggered, the needle protection sleeve (3) acting on the second spring (10),
**characterized in that**
d) the housing (2) comprises a retaining portion, the retaining portion of the housing projecting in the distal direction beyond a distal end of the housing, and the retaining portion of the housing (2) having one or more grooves (2d) or alternatively one or more rails which, in conjunction with a rail or rails (3c) provided on the needle protection sleeve (3) or alternatively in conjunction with a groove or grooves provided on the needle protection sleeve (3), form an anti-rotation element between the housing (2) and the needle protection sleeve (3), and
e) the propulsion element (7; 7') has, on an inner side, an inwardly projecting rib (7c; 7c') or inwardly projecting ribs (7c; 7c') for adjusting a spring tension of the first spring (9), the length of the rib (7c; 7c') or the ribs (7c; 7c') determining a pretensioning of the first spring (9) such that it can dispense the product from the product container (13b) by moving the propulsion element (9) by a dispensing stroke (H_{A}).

2. Autoinjector according to claim 1, **characterized in that** the retaining portion of the housing (2) is sleeve-shaped and is arranged in the interior of the housing (2).

3. Autoinjector according to claim 1 or 2, **characterized in that** the groove (2d) or the rail of the retaining portion of the housing (2) is arranged on an outer lateral surface of the retaining portion of the housing (2).

4. Autoinjector according to any of the preceding claims, **characterized in that** the groove or the rail (3c) of the needle protection sleeve (3) is provided on an inner lateral surface of the needle protection sleeve (3).

5. Autoinjector according to any of the preceding claims, **characterized in that** a stop for limiting the axial movement of the needle protection sleeve (3) in the proximal direction is provided between the sleeve-shaped retaining portion of the housing (2) and the sleeve-shaped housing (2).

6. Autoinjector according to any of the preceding claims, **characterized by** a switching module (8, 15) which is arranged kinematically and/or geometrically between the second spring (10) and the needle protection sleeve (3), the switching module (8, 15) being entrained by the needle protection sleeve (3) in the proximal direction when the needle protection sleeve (3) is moved from its starting position in the proximal direction.

7. Autoinjector according to claim 6, **characterized in that** the switching module (8, 15) has a switching sleeve (15) and a blocking sleeve (8), the blocking sleeve (8) having a unidirectionally acting locking element (8a) which engages in the switching sleeve (15), and the switching sleeve (15), during its movement relative to the housing (2) in the proximal direction, entraining the blocking sleeve (8) via the locking element (8a) and, during its movement relative to the housing (2) in the proximal direction, being moved relative to the blocking sleeve (8) into a further blocking position in which the locking element (8a) blocks a movement of the switching sleeve (15) relative to the blocking sleeve (8) in the proximal direction.

8. Autoinjector according to any of the preceding claims, **characterized in that,** after the product has been dispensed, in particular when the propulsion element (7; 7') has been moved by the dispensing stroke (H_{A}) in the distal direction, the needle protection sleeve (3) can be moved by the second spring (10) relative to the housing (2) in the distal direction, in particular by a needle protection stroke (Hs), into a needle protection position in which the needle protection sleeve (3) is positioned distally above the needle tip of an injection needle (13a) of the product container (13).

9. Autoinjector according to claim 8, **characterized by** a locking element (12a) which locks the needle protection sleeve (3) in its needle protection position relative to the housing (2) against being pushed back in the proximal direction, in particular locks it at least in such a way that the needle tip cannot protrude from the distal end of the needle protection sleeve (3).

10. Autoinjector according to any of the preceding claims, **characterized by** a signal element (11), a signal stop (12b) and the second spring (10), which exerts a spring force acting counter to the dispensing direction on the signal element (11), the signal element (11) being axially fixedly coupled to the propulsion element (7; 7'), so that the signal element (11) is entrained during the movement of the propulsion element (7; 7') in the dispensing direction and the second spring (10) is tensioned, wherein the axially fixed coupling between the signal element (11) and the propulsion element (7; 7') can be released and the signal element (11) can be accelerated counter to the dispensing direction and relative to the propulsion element (7; 7') and/or the housing (2) by means of the second spring (10), wherein the signal element (11) which is decoupled from the axially fixed coupling with the propulsion element (7; 7') and is accelerated by the second spring (10) strikes against the signal stop (12b), as a result of which an acoustic and/or tactile signal is generated.

11. Autoinjector according to claim 10, **characterized in that** the signal element (11) has a first engagement element (11a) which releasably engages in the propulsion element (7; 7'), as a result of which the propulsion element (7; 7') is axially fixedly coupled to the signal element (11), the axially fixed coupling between the propulsion element (7; 7') and the signal element (11) being released when the signal element (11) is out of engagement with the propulsion element (7; 7').

12. Autoinjector according to claim 10 or 11, **characterized in that** the signal stop (12b) is formed by the housing (2) or an element (12) connected to the housing (2) in an at least axially fixed, preferably also rotationally fixed manner, such as a sealing cap (12) which closes the proximal end of the housing (2).

13. Autoinjector according to any of claims 10 to 12, **characterized in that** the signal stop (12b) is arranged along the longitudinal axis (L) of the autoinjector, in particular of the housing (2), in alignment with the signal element (11).

14. A first and a second autoinjector according to any of the preceding claims, **characterized in that** the length of the rib or ribs (7c) of the first autoinjector is different from the length of the rib or ribs (7c') of the second autoinjector, **and in that** the autoinjectors are otherwise identical.

## Revendications

1. Auto-injecteur permettant la distribution d'un produit liquide, en particulier d'un médicament, comprenant :
a) un boîtier (2) et un récipient à produit (13), en particulier une seringue, disposé dans le boîtier (2) et qui présente un piston (13b) qui peut être coulissé, dans lequel le piston (13b) peut être coulissé dans une direction de distribution pour la distribution du produit contenu dans le récipient à produit (13),
b) un élément de traction (7 ; 7') qui agit sur le piston (13b) pendant la distribution de produit, et un premier ressort (9) qui agit sur l'élément de traction (7 ; 7'),
c) une douille de protection d'aiguille (3) qui peut être coulissé dans la direction proximale, en particulier sur une course d'actionnement (H_{B}), à partir de sa position initiale par rapport au boîtier (2) et le long d'un axe longitudinal (L) de l'auto-injecteur pour le déclenchement de la distribution de produit, moyennant quoi un second ressort (10) est tendu et, en particulier, la distribution de produit est déclenchée, dans lequel la douille de protection d'aiguille (3) agit sur le second ressort (10),
**caractérisé en ce que**
d) le boîtier (2) comprend une section de maintien, dans lequel la section de maintien du boîtier fait saillie dans la direction distale au-delà d'une extrémité distale du boîtier, et dans lequel la section de maintien du boîtier (2) présente une ou plusieurs cannelures (2d) ou, alternativement, un ou plusieurs rails qui, en liaison avec le ou les rails (3c) prévus sur la douille de protection d'aiguille (3) ou, alternativement, en liaison avec la ou les cannelures prévues sur la douille de protection d'aiguille (3), forment un blocage de rotation entre le boîtier (2) et la douille de protection d'aiguille (3), et
e) l'élément de traction (7 ; 7') présente, sur un côté intérieur, une nervure (7c ; 7c') faisant saillie vers l'intérieur ou des nervures (7c ; 7c') faisant saillie vers l'intérieur permettant le réglage d'une tension de ressort du premier ressort (9), dans lequel la longueur de la nervure (7c ; 7c') ou des nervures (7c ; 7c') détermine une prétension du premier ressort (9) de telle sorte qu'il peut distribuer le produit hors du récipient à produit (13b) en coulissant l'élément de traction (9) sur une course de distribution (H_{A}).

2. Auto-injecteur selon la revendication 1, **caractérisé en ce que** la section de maintien du boîtier (2) est réalisée en forme de douille et est disposée à l'intérieur du boîtier (2).

3. Auto-injecteur selon la revendication 1 or 2, **caractérisé en ce que** la cannelure (2d) ou le rail de la section de maintien du boîtier (2) est disposé sur une surface extérieure d'enveloppe de la section de maintien du boîtier (2).

4. Auto-injecteur selon l'une des revendications précédentes, **caractérisé en ce que** la cannelure ou le rail (3c) de la douille de protection d'aiguille (3) est prévu sur une surface intérieure d'enveloppe de la douille de protection d'aiguille (3).

5. Auto-injecteur selon l'une des revendications précédentes, **caractérisé en ce qu'une** butée est prévue entre la section de maintien en forme de douille du boîtier (2) et le boîtier (2) en forme de douille pour la limitation du déplacement axial de la douille de protection d'aiguille (3) dans la direction proximale.

6. Auto-injecteur selon l'une des revendications précédentes, **caractérisé par** un module de commutation (8, 15) qui est disposé cinématiquement et/ou géométriquement entre le second ressort (10) et la douille de protection d'aiguille (3), dans lequel le module de commutation (8, 15) est emporté par la douille de protection d'aiguille (3) dans la direction proximale lorsque la douille de protection d'aiguille (3) est coulissée hors de sa position initiale dans la direction proximale.

7. Auto-injecteur selon la revendication 6, **caractérisé en ce que** le module de commutation (8, 15) présente une douille de commutation (15) et une douille d'arrêt (8), dans lequel la douille d'arrêt (8) présente un élément de verrouillage (8a) agissant de manière unidirectionnelle, lequel vient en prise dans la douille de commutation (15), dans lequel la douille de commutation (15), lors de son déplacement par rapport au boîtier (2) dans la direction proximale, emporte la douille d'arrêt (8) par l'intermédiaire de l'élément de verrouillage (8a) et la douille de commutation, lors de son déplacement par rapport au boîtier (2) dans la direction proximale, est coulissée par rapport à la douille d'arrêt (8) dans une autre position d'arrêt dans laquelle l'élément de verrouillage (8a) arrête un déplacement de la douille de commutation (15) par rapport à la douille d'arrêt (8) dans la direction proximale.

8. Auto-injecteur selon l'une des revendications précédentes, **caractérisé en ce que,** après la distribution de produit, en particulier lorsque l'élément de traction (7 ; 7') est coulissé sur la course de distribution (H_{A}) dans la direction distale, la douille de protection d'aiguille (3) peut être coulissée par le second ressort (10) par rapport au boîtier (2) dans la direction distale, en particulier sur une course de protection d'aiguille (Hs) dans une position de protection d'aiguille dans laquelle la douille de protection d'aiguille (3) se trouve en position distale au-dessus de la pointe d'aiguille d'une aiguille d'injection (13a) du récipient à produit (13).

9. Auto-injecteur selon la revendication 8, **caractérisé par** un élément de verrouillage (12a) qui verrouille la douille de protection d'aiguille (3) dans sa position de protection d'aiguille par rapport au boîtier (2) pour l'empêcher de coulisser en sens inverse dans la direction proximale, en particulier la verrouille au moins de telle sorte que la pointe d'aiguille ne peut pas faire saillie hors de l'extrémité distale de la douille de protection d'aiguille (3).

10. Auto-injecteur selon l'une des revendications précédentes, **caractérisé par** un élément de signalisation (11), une butée de signalisation (12b) et le second ressort (10) qui exerce sur l'élément de signalisation (11) une tension de ressort agissant à l'encontre de la direction de distribution, dans lequel l'élément de signalisation (11) est accouplé de manière axialement solidaire à l'élément de traction (7 ; 7'), de sorte que l'élément de signalisation (11) est emporté pendant le coulissement de l'élément de traction (7 ; 7') dans la direction de distribution et que le second ressort (10) est tendu, dans lequel l'accouplement axialement solidaire entre l'élément de signalisation (11) et l'élément de traction (7 ; 7') peut être rompu et l'élément de signalisation (11) peut être accéléré au moyen du second ressort (10) à l'encontre de la direction de distribution et par rapport à l'élément de traction (7 ; 7') et/ou au boîtier (2), dans lequel l'élément de signalisation (11) désaccouplé de l'accouplement axialement solidaire avec l'élément de traction (7 ; 7') et accéléré par le second ressort (10) vient buter contre la butée de signalisation (12b), moyennant quoi un signal acoustique et/ou tactile est créé.

11. Auto-injecteur selon la revendication 10, **caractérisé en ce que** l'élément de signalisation (11) présente un premier élément de mise en prise (11a) qui vient en prise de manière amovible dans l'élément de traction (7 ; 7'), moyennant quoi l'élément de traction (7 ; 7') est accouplé de manière axialement solidaire à l'élément de signalisation (11), dans lequel l'accouplement axialement solidaire entre l'élément de traction (7 ; 7') et l'élément de signalisation (11) est rompu lorsque l'élément de signalisation (11) n'est plus en prise avec l'élément de traction (7 ; 7').

12. Auto-injecteur selon la revendication 10 ou 11, **caractérisé en ce que** la butée de signalisation (12b) est formée par le boîtier (2) ou par un élément (12) relié au moins de manière axialement solidaire, de préférence également solidaire en rotation, au boîtier (2) comme par exemple un capuchon de fermeture (12) qui ferme l'extrémité proximale du boîtier (2).

13. Auto-injecteur selon l'une des revendications 10 à 12, **caractérisé en ce que** la butée de signalisation (12b) est disposée le long de l'axe longitudinal (L) de l'auto-injecteur, en particulier du boîtier (2), en affleurement avec l'élément de signalisation (11).

14. Premier et second auto-injecteur selon l'une des revendications précédentes, **caractérisés en ce que** la longueur de la nervure ou des nervures (7c) du premier auto-injecteur diffère de la longueur de la nervure ou des nervures (7c') du second auto-injecteur, **et en ce que** les auto-injecteurs sont autrement identiques.
